# EUROPEAN PATENT APPLICATION

(11) **EP 1 178 318 A1**
(43) Date of publication of application: **06.02.2002**
(21) Application number: 00402226.5
(22) Date of filing: 03.08.2000
(51) Int. Cl.: G01N 33/68

(54) **A novel class of compounds for the specific detection of polypeptides of interest and their use in detection methods**

(71) Applicant: Hybrigenics S.A., 75012 Paris (FR)
(72) Inventor: Strosberg, Arthur Donny, 75015 Paris (FR); Legrain, Pierre, 75015 Paris (FR)
(74) Representative: Desaix, Anne

(57) **Abstract**

The present invention relates to a novel class of compounds that are useful for the specific detection, labelling, targeting or purification of polypeptides of interest.

The invention also relates to methods for the specific detection, labelling, targeting or purification of polypeptides of interest that make use of the novel class of compounds above as well as to kits specifically designed for carrying out such methods.

## Description

### FIELD OF THE INVENTION

The present invention relates to a novel class of compounds that are useful for the specific detection, labelling, targeting or purification of polypeptides of interest.

The invention also relates to methods for the specific detection, labelling, targeting or purification of polypeptides of interest that make use of the novel class of compounds above as well as to kits specifically designed for carrying out such methods.

Are also encompassed pharmaceutical compositions comprising a compound of the invention or a nucleic acid encoding it.

### BACKGROUND OF THE INVENTION

There is a long standing need in the art for means directed to the specific detection, labelling, targeting or purification of molecules of interest, particularly of polypeptides of interest within a sample.

An important requirement for the design of such detection, labelling, targeting or purification means consists of the availability of a large repertoire of variable molecules which are obtainable with common methods of preparation and within the large population of which must be found and selected those which are specific ligands for a given polypeptide of interest.

To date, the sole technical solution which has been found in order to select compounds belonging to large population of compounds of great diversity and wherein each compound binds specifically to a given ligand, particularly to a given polypeptide of interest, consists in the production of antibodies raised against said ligand of interest, particularly said polypeptide of interest.

These antibodies include polyclonal and monoclonal antibodies and fragments thereof such as Fab and F(ab)'₂ as well as chimeric single chain Fv antibody fragments, antibody fragments obtained through phage display libraries and humanised antibodies.

However, the use of antibodies or antibody fragments for detection purpose is endowed with various drawbacks :
(i) antibodies, including antibody fragments, are complex polypeptides wherein the portion which is involved in the specific recognition of the ligand of interest, typically the protein of interest, is composed of at least one of the three hypervariable regions of both the heavy chain and the light chain of the antibody molecule which is not easy to produce, even with genetic engineering techniques;
(ii) the production of antibodies or antibody fragments containing a complementary determining region (CDR) which recognizes the target molecule in a complex environment with a high specificity for the targeted molecule and in the absence of cross recognition of structurally related domains of non target molecules also present in said complex environment is highly difficult or even impossible to reach;
(iii) the production of antibodies or antibody fragments endowed with a high affinity for a given epitope comprised in the targeted molecule requires a complicated and longworthy selection of the antibodies or antibody fragments of interest among a large initial population of antibodies having various affinities and specificities for the targeted molecule;
(iv) antibodies as well as their fragments are high molecular weight molecules that are not easy to handle for detection purposes, for example in situations wherein a detection step requires the passage of the antibody or the antibody fragment through a cellular membrane, including the membranes of cellular compartments such as nucleus, ribosomes, lysosomes and Golgi apparatus. It is herein reminded that the average molecular weight of an antibody is 150 kDa and the average molecular weight of a (Fab')₂ antibody fragment is of about 100 kDa.
(v) The quality and the quantity of the antibodies produced are dependent of multiple empirical factors including (a) spatial conformation of the antigen and/or the immunogen, (b) the adjuvant properties of the immunisation composition used, (c) the animal to be immunised with the immunisation composition, since it is widely known that the immune response is linked to specific haplotypes of the major histocompatibility complex;
(vi) the obtention of a selected antibody endowed with the suitable properties of affinity and specificity for the desired antigen is highly expensive.

Thus, there is a need in the art for other classes of compounds that recognize specifically a polypeptide of interest and wherein said compounds:
(a) exhibit a high affinity for the target polypeptide ;
(b) possess a high specificity for the targeted polypeptide substantially in the absence of cross-reactions with non-targeted polypeptides even in the context of a complex environment such as for example a biological fluid, a tissue sample or a living cell;
(c ) is easily and reproducibly synthesised, either by a chemical method or by a genetic engineering technique;
(d) has a low molecular weight which confers to such a compound the ability of being easily introduced into a living cell and of migrating within the cell cytoplasm without being prevented to penetrate into anyone of the cell compartments such as the nucleus, ribosomes, Golgi apparatus, lysosomes or chloroplasts.

### SUMMARY OF THE INVENTION

The inventors have now shown that a novel class of compounds termed « Selected Interacting Domain (SID®) » polypeptides satisfy the whole requirements for optimally detecting, labelling, targeting or purifying a polypeptide of interest which are detailed in the previous section entitled « Background of the invention ».

Since these compounds are able to recognize specifically polypeptides, including polypeptides in their natural environment, particularly polypeptides involved in physiological cellular pathways or viral polypeptides expressed in infected cells, the novel class of compounds of the invention may also be used as active ingredients in pharmaceutical compositions.

The invention is primarily directed to the use of a Selected Interacting Domain (SID®) polypeptide or a variant thereof for detecting, labelling, targeting or purifying specifically a polypeptide of interest within a sample.

Thus, the present invention provides for novel marker compounds for the detection, labelling, targeting or purifying a polypeptide of interest as well as methods and devices using the same.

According to one aspect of the invention, a marker compound for the detection, labelling, targeting or purifying a polypeptide of interest within a sample, which marker compound comprises a « Selected Interaction Domain (SID®) polypeptide or a variant thereof is provided.

According to a specific embodiment of a marker compound according to the invention, said marker compound may be chemically synthesised.

According to a second embodiment of said marker compound, this marker compound may be recombinantly produced through genetic engineering techniques.

According to a still further aspect of the invention, methods for the detection, labelling, targeting or purifying of a polypeptide of interest that make use of a marker compound as defined above or alternatively that make use of a « Selection Interacting Domain (SID®) » polypeptide or variant thereof are provided.

In yet a further aspect, the invention also provides for kits for detecting, labelling, targeting, purifying a polypeptide of interest that comprises « Selected Interacting Domain (SID®) polypeptide or a variant thereof.

In still a further aspect, the invention provides for a device or an apparatus for the detection of a polypeptide or a plurality of polypeptides of interest wherein said device or apparatus comprises a substrate onto which a « Selected Interacting Domain (SID®) » polypeptide or a plurality of « Selected Interacting Domain (SID®) » polypeptides or variants thereof is (are) immobilised.

In yet another aspect, the invention also relates to methods for labelling a polypeptide of interest which make use of a Selected Interacting Domain (SID®) polypeptide or a variant thereof, as well as method for purifying a polypeptide of interest using the same.

The present invention also concerns pharmaceutical compositions comprising a pharmaceutically effective amount of a Selected Interacting Domain (SID®) polypeptide or a variant thereof, or a nucleic acid or a recombinant expression vector encoding said Selected Interacting Domain (SID®) polypeptide or said variant thereof, more particularly when said Selected Interacting Domain (SID®) polypeptide or said variant thereof has the ability to bind specifically to a viral or bacterial protein.

The various properties listed above of the novel class of compounds of the invention allow their use *in vitro* or *in vivo* for interfering with the naturally occurring binding between two proteins involved in a functional physiological pathway, thus disturbing the physiology of the cell host concerned, including causing the cell death of said cell host.

Therefore, the present invention is also directed to a pharmaceutical composition comprising a Selected Interacting Domain polypeptide (SID®) or a polynucleotide encoding it.

Throughout this application, various publications, patents and published Patent Applications are cited. The disclosures of these publications, patents and published patent specifications referenced in this application are hereby incorporated by reference into the present disclosure to more fully describe the state of the art to which this invention pertains.

### BRIEF DESCRIPTION OF THE FIGURES

Fig.**1** is a restriction map of the plasmid pAS2ΔΔ which may be used for producing a recombinant « Selected Interacting Domain (SID®) » polypeptide or a recombinant marker compound of the invention.

Fig. **2** is a restriction map of the plasmid pACTII which may be used for producing a recombinant « Selected Interacting Domain (SID®) ».

Fig. **3** is a restriction map of the plasmid pUT18 which may be used for producing a recombinant « Selected Interacting Domain (SID®) ».

Fig. **4** is a restriction map of the plasmid pUT18C which may be used for producing a recombinant « Selected Interacting Domain (SID®) ».

Fig. **5** is a restriction map of the plasmid pT25 which may be used for producing a recombinant « Selected Interacting Domain (SID®) ».

Fig. **6** is a restriction map of the plasmid pKT25 which may be used for producing a recombinant « Selected Interacting Domain (SID®) ».

Fig.**7** is a schematic representation of the SID® identification method. In this figure, the « full length prey protein » is the open reading frame where the identified prey polypeptides are included. The « Selected Interacting Domain (SID®) » is determined by comparison of every prey polypeptide fragment.

### DETAILED DESCRIPTION OF THE INVENTION.

The present invention provides for a novel class of marker compounds that bind specifically to a polypeptide of interest and wherein said compound comprises a « Selected Interacting Domain (SID®) » polypeptide that binds specifically to the polypeptide of interest.

### SELECTED INTERACTING DOMAIN (SID®) POLYPEPTIDES AND METHODS FOR THEIR PREPARATION.

A selected interacting domain polypeptide that binds specifically to a polypeptide of interest is the result of a two-step screening procedure, wherein :
1) the first step consists of selecting and characterizing a collection of nucleic acids (prey nucleic acids) encoding polypeptides which bind specifically to a given bait polypeptide of interest; and
2) the second step of the two-step procedure consists of comparing the nucleotide sequences of said collection of selected prey nucleic acids and determining one or several polynucleotides common to said all prey nucleic acids.

Every nucleotide sequence which is common to said all selected prey nucleic acids consists of the polynucleotide encoding the « Selected Interacting Domain (SID®) » polypeptide which binds with a high specificity with the bait polypeptide of interest.

### Step 1) Selecting prey nucleic acids

### a) Yeast two-hybrid system.

The first step of selecting a collection of nucleic acids encoding polypeptides which binds specifically to the bait polypeptide may be carried out through a yeast two-hybrid system. The yeast two-hybrid system is designed to study protein-protein interactions *in vivo,* and relies upon the fusion of a bait protein to the DNA binding domain of the yeast Gal4 protein.

Most preferably, the first step of the procedure for selecting a Selected Interacting Domain (SID®) polynucleotide encoding a Selected Interacting Domain (SID®) polypeptide is the two hybrid screening system described by Fromont-Racine et al. (1997) or the method described by Flajolet et al. (2000). The yeast two-hybrid system utilises hybrid proteins to detect protein-protein interactions by means of direct activation of a reporter gene expression. In essence , the nucleic acids encoding the two putative protein partners, respectively the bait polypeptide of interest and the prey polypeptide, are genetically fused to the DNA-binding domain of a transcription factor and to a transcriptional activation domain, respectively. A productive interaction between the two proteins of interest will bring the transcriptional activation domain of an adjacent reporter gene giving a screenable phenotype. The transcription can be activated through the use of two functional domains of a transcription factor, respectively a domain that recognizes and binds to specific site of the DNA and a domain that is necessary for activation (Keegan et al., 1986).

In one embodiment of a two hybrid assay which is carried out at step 1) of the procedure for selecting a Selected Interaction Domain (SID®) polypeptides, the nucleotide sequence encoding the bait polypeptide of interest is fused to a polynucleotide encoding the DNA binding domain of the Gal4 protein, the fused nucleotide sequence being inserted in a suitable expression vector such as plasmids PAS2 or pACTII (commercialized by Clontech) or the vector pAS2ΔΔ (Fromont-Racine et al.,1997).

Then, a genomic DNA library (for prokaryotic DNA) or a cDNA library (for prokaryotic and eukaryotic DNA) is constructed in a specially designed vector, such that every DNA or cDNA insert is fused to a nucleotide sequence in the vector that encodes the transcription of domain of the Gal4 protein. Preferably, the vector used is the pACT vector. The polypeptides encoded by the nucleotide inserts of the genomic DNA or cDNA library thus prepared are termed « prey » polypeptides.

A third vector contains a detectable marker gene, such as β-galactosidase gene or chloramphenicol acetyl transferase (CAT) gene that is placed under the control of a regulation sequence that is responsive to the binding of a complete Gal4 protein containing both the transcription or activation domain and the DNA binding domain, such as described in Fromont-Racine et al. (1997) or Flajolet et al. (2000).

As an illustrative example, the collection of nucleic acid inserts contained in the collection of clones containing the genomic DNA or cDNA library previously prepared are used to transform a first yeast strain, such as the Y187 *Saccharomyces cerevisiae* strain (phenotype:MATα, GaI4Δ, gal80Δ, ade2-101, His3, Leu2-3, -112 Trp1-901, Ura3-52, URA3::UASGAL1-LacZ Met).

The nucleic acid encoding the bait polypeptide of interest is inserted in an appropriate vector, such as plasmid pAS2ΔΔ, said vector being used to transform a second yeast strain which may be the CG1945 (MATa Gal4-542 Gal180-538, Ade2-101, His3*200, Leu2-3, -112 Trp1-901 Ura3-52, Lys2-801, URA3::GAL4 17Mers (X3)-CyC1TATA-LacZ LYS2::GAL1 UAS-GAL1TATA-His3 CYH^{R}).

Then , the two yeast strains are mated to obtain a collection of mated cells.

The clones derived from the collection of mated cells above which are positive in an X-Gal overlay assay are those for which an interaction between the recombinant bait polypeptide and a polypeptide encoded by a nucleic acid insert originating from the genomic or the cDNA library has occurred.

In a further step, the prey nucleic acid inserts contained in the positively selected clones are sequenced.

### b) Bacterial two-hybrid system

A bacterial two-hybrid method of the invention may be performed by the one skilled in the art according to the teachings of KARIMOVA et al. (1998).

The first step of selecting a collection of nucleic acids encoding polypeptides which binds specifically to the bait polypeptide may also be carried out through a bacterial two-hybrid system.

According to such bacterial two-hybrid system, bacterial cell clones, preferably *Escherichia coli* cells, are transformed with a plasmid containing a bit polynucleotide encoding a bait polypeptide.

Then, plasmids containing a DNA insert are provided by rescuing the plasmids obtained from the collection of yeast clones containing the genomic DNA or cDNA library which are described in the previous section entitled « Yeast two-hybrid system ». For example, the plasmid rescue may be carried out according to the following steps:
(i) extracting plasmid DNA contained in the collection of yeast clones obtained as disclosed in the previous sections, by using a conventional DNA extraction buffer and a phenol: chloroform: isoamyl alcohol (25:24:1) before centrifuging;
(ii) transferring a desired volume of the supernatant obtained at the end of step (i) to a sterile Eppendorf tube and add a precipitation buffer (ethanol/NH₄Ac) before centrifuging and resuspending the pellet after washing in ethanol;
(iii) transforming *Escherichia coli* cells (e.g. *Escherichia coli* cells of strain NC 1066) which have been rendered electrocompetent with a desired volume (e.g. 1 µl) of the yeast plasmid DNA extract obtained at step (ii) by electroporation;
(iv) collecting the transformed *Escherichia coli* cells.

Alternatively, a collection of *Escherichia coli* cell clones containing a collection of genomic DNA or cDNA inserts may be obtained by constructing the DNA library directly in the bacterial cell, such as disclosed in Flajolet et al. (2000).

Then, the bacterial recombinant cells which have been transformed both with a plasmid containing a bait polynucleotide encoding a bait polypeptide and a plasmid containing a prey polynucleotide encoding a prey polynucleotide is cultivated on a selective medium.

Then, recombinant cell clones capable of growing on said selective medium are selected and the DNA inserts of the plasmids containing therein are sequenced.

By bacterial two-hybrid system is generally intended a method that usually makes use of at least one reporter gene, the transcription of which is activated when a prey polypeptide and a bait polypeptide produced by the recombinant cell due to the triggering of the transcription of said at least one reporter gene when both the specific domain contained in one prey polypeptide and the complementary domain contained in the bait polypeptide are binding one to the other.

### Step 2:determination of the nucleic acid sequences encoding a Selected Interacting Domain (SID®) polypeptide which binds specifically to a bait polypeptide of interest.

This is the second step of the two steps procedure defined above.

A nucleic acid sequence encoding a Selected Interacting Domain (SID®) polypeptide may be generally defined as a nucleic acid sequence which consists of an overlapping portion between a set of polynucleotides wherein each polynucleotide of said set of polynucleotides encodes a first polypeptide (e.g. a prey polypeptide) which binds specifically to a second polypeptide (e.g. a bait polypeptide).

Preferably, each polynucleotide of the set of overlapping polynucleotides cited above has been selected according to a two hybrid system method.

Most preferably, each of the polynucleotide belonging to the set of polynucleotides cited above has been selected according to a yeast two-hybrid system or a bacterial to hybrid system which is disclosed specifically in the description of step 1 above.

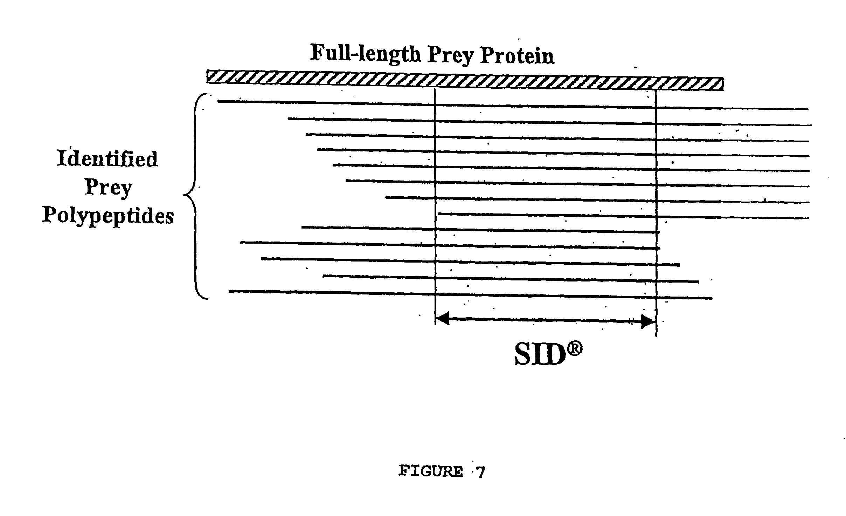

As depicted in figure **7**, an illustrative example for the determination of the nucleic acid sequence encoding the Selecting Interacting Domain (SID®) polypeptide comprises the steps of:
a) carrying out a sequence alignment between the collection of the prey nucleic acid sequences contained in the different positively selected yeast clones wherein an interaction has occurred between the polypeptide encoded by the prey nucleic acid and the recombinant bait polypeptide of interest;
b) determining the largest shared overlapping nucleic acid sequence between the whole collection of prey nucleic acid sequences aligned in step a), wherein said largest shared overlapping nucleic acid sequence encodes the Selected Interacting Domain (SID®) polypeptide.

### Variants of a Selected Interacting Domain (SID®) polypeptide

As intended herein, a variant of a Selected Interacting Domain (SID®) polypeptide may be either a variant polypeptide of the Selected Interacting Domain (SID®) polypeptide or a polypeptide which is encoded by a nucleic acid variant of the polynucleotide encoding said Selected Interacting Domain (SID®) polypeptide.

Variants of polynucleotides encoding a polypeptide variant of a Selected Interacting Domain (SID®) polypeptide, as the term is used herein, are polynucleotides that differ from the reference polynucleotide. A variant of a polynucleotide may be a naturally occurring variant such as a naturally occurring allelic variant, or it may be a variant that is not known to occur naturally. Such none-naturally occurring variants of the reference polynucleotide may be made by mutagenesis techniques, including those applied to polynucleotides, cells or organisms.

Generally, differences are limited so that the nucleotide sequences of the reference and the variant are closely similar overall and, in many regions, identical.

Variants of polynucleotides according to the invention include, without being limited to, nucleotide sequences which are at least 95% identical after optimal alignment to the reference polynucleotide encoding the reference Selected Interacting Domain (SID®) polypeptide, preferably at least 96%, 97%, 98% and most preferably at least 99% identical to the reference polynucleotide.

Nucleotide changes present in a variant polynucleotide may be silent, which means that they do not alter the aminoacid encoded by the reference polynucleotide.

However, nucleotide changes may also result in aminoacid substitutions , additions, deletions, fusions and truncations in the Selected Interacting Domain (SD®) polypeptide encoded by the reference sequence.

The substitutions, deletions or additions may involve one or more nucleotides. Alterations may produce conservative or non-conservative aminoacid substitutions, deletions or additions.

Most preferably, the variant of a Selected Interacting Domain (SID®) polypeptide encoded by a variant polynucleotide possess a higher affinity of binding to its protein or polypeptide counterpart, against which it has been initially selected as described above.

In another preferred embodiment, the variant of a Selected Interacting Domain (SID®) polypeptide which is encoded by a variant polynucleotide of the invention possess a higher specificity of binding to its counterpart polypeptide or protein than the reference Selected Interacting Domain (SID®) polypeptide.

A variant of a Selected Interacting Domain (SID®) polypeptide according to the invention may be (1) one in which one or more of the aminoacid residues are substituted with a conserved or a non-conserved aminoacid residue and such substituted aminoacid residue may or may not be one encoded by the genetic code, or (2) one in which one or more of the aminoacid residues includes a substituent group.

In the case of an aminoacid substitution in the aminoacid sequence of a Selected Interacting Domain (SID®) polypeptide according to the invention, one or several-consecutive or nonconsecutive-aminoacids are replaced by « equivalent » aminoacids. The expression « equivalent» aminoacid is used herein to designate any aminoacid that may be substituted for one of the aminoacids belonging to the native Selected Interacting Domain (SID®) polypeptide structure without decreasing the binding properties of the corresponding peptides to their counterpart polypeptide or protein, as regards the reference Selected Interacting Domain (SID®) polypeptide.

These equivalent aminoacids may be determined either by their structural homology with the initial aminoacids to be replaced, by the similarity of their net charge or of their hydrophobicity.

By an equivalent aminoacid according to the present invention is also meant the replacement of a residue in the L-form by a residue in the D-form or the replacement of a glutamic acid residue by a pyroglutamic acid compound. The synthesis of peptides containing at least one residue in the D-form is, for example, described by KOCH (1977). A specific embodiment of a variant of a Selected Interacting Domain (SID®) polypeptide according to the invention includes, but is not limited to, a peptide molecule which is resistant to proteolysis, such as a peptide in which the -CONH- peptide bond is modified and replaced by a (- CH₂NH-) reduced bond, a (-NHCO-) retroinverso bond, a (-CH₂-O-) methylene-oxy bond, a (-CH₂-S-) thiomethylene bond, a (-CH₂CH₂-) carba bond, a (-CO-CH₂) hydroxyethylene bond, a (-N-N-) bond or also a -CH=CH bond.

In a first preferred embodiment, a variant of a Selected Interacting Domain (SID®) polypeptide possesses a higher affinity for binding onto its counterpart protein or polypeptide as regards the reference Selected Interacting Domain (SID®) polypeptide.

In a second preferred embodiment, a variant of a Selected Interacting Domain (SID®) polypeptide has a higher affinity for its counterpart polypeptide or protein as regards the reference Selected Interacting Domain (SID®) polypeptide.

### MARKER COMPOUNDS OF THE INVENTION

It has now been shown by the inventors that the Selected Interacting Domain (SID®) polypeptides or variants thereof defined in the section above and which bind specifically to a polypeptide of interest (e.g. a bait polypeptide) are useful as reagents for detecting, labeling, targetting or purifying specifically a polypeptide of interest within a sample, since the SID® polypeptides possess properties that have never been reached using conventional detection compounds, such as those of an antibody or an antibody fragment.

Firstly, the SID® polypeptides possess a high specificity of binding to the polypeptide of interest, since a SID® polypeptide consists of a portion of a larger polypeptide which binds in a highly specific manner to the polypeptide of interest in the natural environment within the prokaryotic or the eukaryotic cell;

Secondly, the SID® polypeptide generally has a low molecular weight, generally from 1 kDa, and are thus easy to produce, on the one hand, and, on the other hand, can be easily introduced within a cell when the detection of the localisation or of the expression of the polypeptide of interest is sought. Moreover, the small size of a SID® polypeptide allows its passage through inner cell barriers such as the nucleus membrane, or the membranes surrounding the different cell organites.

Thus, a first object of the invention consists of a marker compound wherein said compound comprises :
a) a Selected Interacting Domain (SID®) polypeptide or a variant thereof that binds specifically to the polypeptide of interest; and
b) a detectable molecule bound thereto.

Such a marker compound is primarily useful for detecting, labeling or targetting a polypeptide of interest, for example a polypeptide of interest contained in a sample.

A detectable molecule according to the invention comprises, or alternatively consists of , any molecule which produces or can be induced to produce a signal. The detectable molecule can be a member of the signal producing system that includes the signal producing means

The detectable molecule may be isotopic or non isotopic. By way of example and not limitation, the detectable molecule can be part of a catalytic reaction system such as enzymes, enzyme fragments, enzyme substrates, enzyme inhibitors, co-enzymes, or catalysts. Part of a chromogen system such as fluorophores, dyes, chemiluminescers, luminescers, or sensitizers. A dispersible particle that can be nonmagnetic or magnetic, a solid support, a liposome, a ligand, a receptor, a hapten radioactive isotope, and soforth.

It must be generally understood that the whole embodiments disclosed in the present specification involving a Selected Interacting Domain (SID®) polypeptide is straightfully applied also to any variant thereof.

### Fluorescent detectable molecules

In one aspect of the marker compound according to the invention, the detectable molecule consists of a fluorescent molecule. Fluorescent moieties which are frequently used as labels are for example those described by Ichinose et al. (1991). Other fluorescent detectable molecules are fluorescing isothiocyanate (FITC) such as described by Shattil et al. (1987) or by Goding et al. (1986). The fluorescent detectable molecule may also comprise a phycoerythrin as taught by Goding et al. (1986), and Shattil et al. (1985). Other examples of fluorescent detectable molecules suitable for use as labels of a marker compound according to the invention are rhodamine isothiocyanate, dansyl chloride and XRITC.

Other fluorescent detectable molecules usable as labels for a marker compound according to the invention encompasses fluorescers falling into a variety of categories having certain primary functionalities, such as those including 1- and 2-aminonaphtalene, p,p-diaminostilbenes, pyrenes, quaternary phenanthridine salts, 9-aminoacridines, p,p'-diaminostylbenes imines, anthracenes, oxacarboxyanine, merocyanine, 3-aminoequilenin, perylene, bis-benzoxazole, bis-p-oxazolyl benzene, 1,2-benzophenazine, retinol, bis-3-aminopyridinium salts, hellebrigenin, tetracycline , sterophenol, benzimidazolylphenylamine, 2-oxo-3-chromen, indole, xanthene, 7-hydroxycoumarin, 4,5-benzymidazoles, phenoxazine or flavin. Exemplary fluorescent molecules are enumerated in US Patent N°4,318,707, columns 7 and 8, the disclosure of which is incorporated herein by reference.

Another fluorescent detectable molecule consists of the green fluorescent protein (GFP) of the jelly fish *Aequorea victoria,* and their numerous fluorescent protein derivatives.

The one skilled in the art may advantageously refer to the articles of CHALFIE et al. (1994) and of HEIM et al. (1994) which discloses the uses of GFP for the study of gene expression and protein localisation. The one skilled in the art may also refer to the article of Rizzuto et al. (1995), which discusses the use of wild-type GFP as a tool for visualising subcellular organelles in cells, to the article of KAETHER and GERDES (1995), which reports the visualisation of protein transport along the secretary passway using wild-type GFP, the article of HU and CHENG (1995), which relates to the expression of GFP in plant cells and also to the article of Davis et al. (1995) which discloses the GFP expression in drosophilia embryos. For the use of several fluorescent variants of GFP, the one skilled in the art may refer to the article of Delagrave et al. (1995), as well as to the article of Heim et al. (1995). DNA encoding GFP is available commercially, for example from Clontech in Palo Alto, California, USA. The one skilled in the art may use also humanized GFP genes such as those described in the US Patent N°6,020,192 and also the GFP protein disclosed in the US Patent N°5,941,084.

Another fluorescent protein that may be used in a marker compound according to the invention consists of the yellow fluorescent protein (YFP).

A further suitable fluorescent protein consists of the luciferase protein.

### Detectable molecules exhibiting a catalytic activity

In another embodiment of a detectable molecule included in a marker compound according to the invention, said detectable molecule is endowed with a catalytic activity and may thus consists of enzymes and catalytically active enzyme fragments. Some enzymatic labels are described in US Patent N°3,654,090. Such enzymes may be for example horse radish peroxydase (HRP), alkaline phosphatase or glutathione peroxydase which are well known from the one skilled in the art.

Enzymes, enzyme fragments, enzyme inhibitors, enzyme substrates, and other components of enzyme reaction systems can be used as detectable molecules. Where any of these components is used as a detectable molecule, a chemical reaction involving one of the components is part of the signal producing system.

Coupled catalysts can also involve an enzyme with a non-enzymatic catalyst. The enzyme can produce a reactant, which undergoes a reaction catalysed by the non-enzymatic catalyst or the non-enzymatic catalyst may produce a substrate (including co-enzymes) for the enzyme. The one skilled in the art may advantageously refer to the US Patent N°4,160 645 which disclose a white variety of non enzymatic catalysts, which may be employed, the appropriate portions of which are incorporated therein by reference.

The enzyme or co-enzyme employed provides the desired amplification by producing a product, which absorbs light, e.g., a tye, or emits lights upon irradiation, e.g., a fluorescer. Alternatively, the catalytic reaction can lead to direct light emission, e.g., chemiluminescence. A large number of enzymes and co-enzymes for providing such products are described in the US Patents N°4,275,149, columns 19 to 23 and N°4,318,980, columns 10 to 14 which disclosures are incorporated herein by reference.

A number of enzyme combinations are set forth in US Patent N°4,275,149, columns 23 to 28 which disclosures are incorporated herein by reference.

When a single enzyme is used as the detectable molecule, or alternatively as comprised in the detectable molecule, such enzymes may find use are hydrolases, transferases, lyases, isomerases, ligases or synthetases and oxydoreductases.

Alternatively, luciferases may be used such as firefly luciferase and bacterial luciferase.

Primarily, the enzymes of choice, based on the I.U.B. classification are: (i) class 1. Oxydoreductases and (ii) class 3. Hydrolases. Most preferred oxydoreductases are (i) dehydrogenases of class 1.1, more particularly 1.1.1, 1.1.3. and 1.1.99 and (ii) peroxydases in class 1.11. of the hydrolases, particularly class 3.1., more particularly 3.1.3 and class 3.2, more particularly 3.2.1. are preferred.

Illustrative dehydrogenases include malate dehydrogenase, glucose-6-phosphate dehydrogenase and lactate dehydrogenase. Of the oxydases, glucose oxydases is exemplary. Of the peroxydases, horse radish peroxydase is illustrative. Of the hydrolases, alkaline phosphatases, β-glucosydase and lysozyme are illustrative.

### Chemiluminescent detectable molecules

The detectable molecule comprised within the marker compound according to the invention may also consist in a chemiluminescent moiety. The chemiluminescent source involves a compound, which becomes electronically excited by a chemical reaction and may emit light which serves at as the detectable signal or donates energy to a fluorescent acceptor.

A diverse number of families of compounds have been found to provide chemiluminescent under a variety of conditions. When family of compounds is 2,3-dihydro-1,4-phtalazinedinone. The most utilised compound is luminol, which is the 5-amino analogue of the compound above. Other members of the family include the 5-amino-6,7,8-trimethoxy-and the dimethylamine-[ca]benzo analogue. These compounds can be made to luminance with alkaline hydrogen peroxyde or calcium hypochlorite and base.

Another family of compounds is the 2,4,5-triphenylimidazoles, with lophine as the common name for the parent product. Chemiluminescent analogues include para-dimethylamino- and paramethoxy-substituents. Chemiluminescents may also be obtained with geridinium esters, dioxetanes and oxalates, usually oxalyl active esters, e.g., p-nitrophenyl and a peroxide, e.g., hydrogen peroxide, under basic conditions. Alternatively, luciferins may be used in conjunction with luciferase or lucigenins.

### Radioactive detectable molecules

In a further embodiment of a detectable molecule comprised in a marker compound according to the invention, said detectable molecule is radio-actively labelled such as with **[**^{**3**}**H], [**^{**32**}**P], [and [**^{**125**}**I].**

### Colloidal metal detectable molecules

In still a further embodiment, the detectable molecule comprised in a marker compound according to the invention may include a colloïdal metal particle. Colloïdal metals have been employed in immuno assays previously. Mostly, they consisted of either colloïdal iron or gold. The one skilled in the art may advantageously refer to the articles of Horisberger (1981) and Martin et al. (1990). In other case, the metals are chosen for their colour, i.e., their presence is determined by their colour or electron density under an electron microscope. Both the colour and electron density are directly proportional to the mass of the metal colloïd.

### STRUCTURE OF THE MARKER COMPOUNDS OF THE INVENTION

In a first preferred embodiment of a marker compound of the invention, the detectable molecule is covalently bound to the Selected Interacting Domain (SID®) polypeptide or a variant thereof.

According to this specific embodiment, detectable molecules comprising fluorescent proteins such as GFP and YFP, enzymes or enzyme fragments such as alkaline phosphatase, glutathion peroxydase and horse radish peroxydase, chemiluminescent molecules, radioactive labels or colloïdal metal particles will be preferred.

General methods that may be used by the one skilled in the art for covalently binding the detectable molecules to the Selected Interacting Domain (SID®) polypeptide are described in the numerous bibliographic references related to the preparation of the antibody conjugates used for carrying out immunoassays.

In a second preferred embodiment of a marker compound according to the invention, the detectable molecule is non-covalently bound to the Selected Interacting Domain (SID®) polypeptide or a variant thereof.

In a first preferred aspect of this second preferred embodiment, the detectable molecule consists of an antibody directed specifically against the Selected Interacting Domain (SID®) polypeptide or a variant thereof.

The antibodies directed specifically against the Selected Interacting Domain (SID®) polypeptide or a variant thereof may be indifferently radioactivity or non radioactivity labelled.

Antibodies directed against a SID® polypeptide may be prepared from hybridomas according to the technique described by Kohler and Milstein in 1975. The polyclonal antibodies may be prepared by immunization of a mammal, especially a mouse or a rabbit, with the SID® polypeptide that is combined with an adjuvant of immunity, and they by purifying of the specific antibodies contained in the serum of the immunized animal on a affinity chromatography column on which has previously been immobilized the polypeptide that has been used as the antigen.

Antibodies directed against a SID® polypeptide may also be produced by the trioma technique and by the human B-cell hybridoma technique (Kozbor et al., 1983).

Antibodies directed to a SID® polypeptide include, chimeric single chain Fv antibody fragments (US Patent N° US 4,946,778, Martineau et al., 1998), antibody fragments obtained through phage display libraries (Ridder etal., 1995) and humanized antibodies (Reinmann et al., 1997; Leger et al., 1997) . Also, transgenic mice, or other organisms such as other mammals , may be used to expresse antibodies, including or example, humanized antibodies directed against a SID® polypeptide of the invention, or a fragment or variant thereof.

In a third preferred embodiment of a marker compound according to the invention, said marker compound is further characterized in that :
a) the Selected Interacting Domain (SID®) polypeptide is covalently bound to a first ligand; and
b) the detectable molecule comprises a second ligand which binds specifically to the first ligand.

According to this third preferred embodiment, the first ligand may be biotin, in which case the second ligand consists of streptavidin.

### NUCLEIC ACIDS OF THE INVENTION.

The present invention also pertains to a nucleic acid encoding a Selected Interacting Domain (SID®) polypeptide or a variant thereof.

The present invention also relates to a nucleic acid encoding a marker compound as defined above.

Most preferred nucleic acids encompassed by the invention include polynucleotides that encode a marker compound wherein the Selected Interacting Domain (SID®) polypeptide or a variant thereof is covalently bound to the detectable molecule and wherein the detectable molecule consists itself of a polypeptide.

In a first preferred embodiment of a nucleic acid according to the invention, said nucleic acid encodes for a Selected Interacting Domain (SID®) polypeptide which is fused to a fluorescence protein, such as GFP and YFP.

In a second preferred embodiment of a nucleic acid according to the invention, said nucleic acid encodes for a Selected Interacting Domain (SID®) polypeptide which is fused to a polypeptide endowed with a catalytic activity, such as an enzyme or an enzymatically active enzyme fragment, like alkaline phosphatase, glutathione peroxydase and horse radish peroxydase.

For the purposes of the present description, the expression "nucleotide sequence" may be used to designate either a polynucleotide or a nucleic acid. The expression "nucleotide sequence" covers the genetic material itself and is therefore not restricted to the information relating to its sequence.

The terms "nucleic acid", "polynucleotide", "oligonucleotide" or "nucleotide sequence" cover RNA, DNA, gDNA or cDNA sequences or alternatively RNA/DNA hybrid sequences of more than one nucleotide, either in the single-stranded form or in the duplex, double-stranded form.

A "nucleic acid" is a polymeric compound comprised of covalently linked subunits called nucleotides. Nucleic acid includes polyribonucleic acid (RNA) and polydeoxyribonucleic acid (DNA), both of which may be single-stranded or double-stranded. DNA includes cDNA, genomic DNA, synthetic DNA, and semi-synthetic DNA. The sequence of nucleotides that encodes a protein is called the sense sequence or coding sequence.

The term "nucleotide" designates both the natural nucleotides (A, T, G, C) as well as the modified nucleotides that comprise at least one modification such as (1) an analogue of a purine, (2) an analogue of a pyrimidine, or (3) an analogous sugar, examples of such modified nucleotides being described, for example, in the PCT application No. WO 95/04 064.

Preferably, anyone of the nucleic acid or the polypeptides encompassed by the invention is under a purified or an isolated form.

The term "isolated" for the purposes of the present invention designates a biological material (nucleic acid or protein) which has been removed from its original environment (the environment in which it is naturally present).

For example, a polynucleotide present in the natural state in a plant or an animal is not isolated. The same polynucleotide separated from the adjacent nucleic acids in which it is naturally inserted in the genome of the plant or animal is considered as being "isolated".

Such a polynucleotide may be included in a vector and/or such a polynucleotide may be included in a composition and remains nevertheless in the isolated state because of the fact that the vector or the composition does not constitute its natural environment.

The term "purified" does not require the material to be present in a form exhibiting absolute purity, exclusive of the presence of other compounds. It is rather a relative definition.

A polynucleotide is in the "purified" state after purification of the starting material or of the natural material by at least one order of magnitude, preferably 2 or 3 and preferably 4 or 5 orders of magnitude.

"Isolated polypeptide" or "isolated protein" is a polypeptide or protein which is substantially free of those compounds that are normally associated therewith in its natural state (e.g., other proteins or polypeptides, nucleic acids, carbohydrates, lipids). "Isolated" is not meant to exclude artificial or synthetic mixtures with other compounds, or the presence of impurities which do not interfere with biological activity, and which may be present, for example, due to incomplete purification, addition of stabilisers, or compounding into a pharmaceutically acceptable preparation.

In a preferred embodiment, a nucleic acid encoding a marker compound of the invention comprises a DNA coding sequence which is transcribed and translated into said marker compound in a cell *in vitro* or *in vivo* when placed under the control of appropriate regulatory sequences. The boundaries of the coding sequence are determined by a start codon and a translation stop codon. A coding sequence can include, but is not limited to:
- prokaryotic sequences, for example when both the Selected Interacting Domain (SID®) nucleic acid and the nucleic acid fused thereto which encodes the detectable molecule are of prokaryotic origin;
- eukaryotic sequences, when both the Selected Interacting Domain (SID®) nucleic acid and the nucleic acid fused thereto which encodes for the detectable molecule originate from a eukaryotic organisms (e.g., a yeast or a mammal);
- prokaryotic and eukaryotic sequences, for example when the Selected Interacting Domain (SID®) nucleic acid originates from an eukaryotic host organism and the nucleic acid encoding the detectable molecule originates from a prokaryotic host organism.

If the coding sequence is intended for expression in an eukaryotic cell, a polyadenylation signal and transcription termination sequence will usually be located 3' to the coding sequence.

In a most preferred embodiment of a nucleic acid sequence according to the invention, said nucleic acid sequence include a regulatory region which is functional in the host organism within which the expression of said nucleic acid sequence is sought, wherein said regulatory region comprises a promoter sequence.

"Regulatory region" means a nucleic acid sequence which regulates the expression of a nucleic acid. A regulatory region may include sequences which are naturally responsible for expressing a particular nucleic acid (a homologous region) or may include sequences of a different origin (responsible for expressing different proteins or even synthetic proteins). In particular, the sequences can be sequences of eukaryotic or viral genes or derived sequences which stimulate or repress transcription of a gene in a specific or non-specific manner and in an inducible or non-inducible manner. Regulatory regions include origins of replication, RNA splice sites, enhancers, transcriptional termination sequences, signal sequences which direct the polypeptide into the secretary pathways of the target cell, and promoters.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced and translated into the protein encoded by the coding sequence.

Expression of a polypeptide of the invention may be controlled by any promoterlenhancer element known in the art, but these regulatory elements must be functional in the host selected for expression. Promoters which may be used to control the expression of the nucleic acid sequence encoding a polypeptide according to the invention include, but are not limited to, the SV40 early promoter region (Benoist and Chambon, 1981), the promoter contained in the 3' long terminal repeat of Rous sarcoma virus (Yamamoto, et al., 1980), the Herpes thymidine kinase promoter (Wagner et al., 1981), the regulatory sequences of the metallothionein gene (Brinster et al., 1982); prokaryotic expression vectors such as the b-lactamase promoter (Villa-Koumaroff, et al., 1978), or the *tac* promoter (DeBoer, et al., 1983; see also "Useful proteins from recombinant bacteria" in Scientific American, 1980, 242:74-94); promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., 1984; MacDonald, 1987); insulin gene control region which is active in pancreatic beta cells (Hanahan, 1985), immunoglobulin gene control region which is active in lymphoid cells (Grosschedl et al., 1984; Adams et al., 1985;), mouse mammary tumor virus control region which is active in testicular, breast, lymphoid and mast cells (Leder et al., 1986), albumin gene control region which is active in liver (Pinkert et al., 1987), alpha-fetoprotein gene control region which is active in liver (Krumlauf et al., 1985;), alpha 1-antitrypsin gene control region which is active in the liver (Kelsey et al., 1987) beta-globin gene control region which is active in myeloid cells (Mogram et al., 1985; Kollias et al., 1986), myelin basic protein gene control region which is active in oligodendrocyte cells in the brain (Readhead et al., 1987), myosin light chain-2 gene control region which is active in skeletal muscle (Sani, 1985), and gonadotropic releasing hormone gene control region which is active in the hypothalamus (Mason et al., 1986).

Most preferably, the regulatory region or the promoter sequence included in a nucleic acid encoding a marker compound of the invention is selected to be fully functional in the cell host within which the expression of the marker compound is sought.

For example, if the Selected Interacting Domain (SID®) polypeptide which is part of a recombinant marker of the invention was determined to interact or bind specifically with a protein which is naturally expressed within a mammal cell (e.g. a given transcription factor expressed in human cells), then the regulatory region or the promoter sequence will be selected among the sequences which are active in mammalian cells (e.g.in human cells).

### VECTORS OF THE INVENTION

The nucleic acids coding for a Selected Interacting Domain (SID®) polypeptide or a variant thereof, or for a marker compound of the invention and which are defined in the section above can be inserted into an appropriate expression vector, i.e., a vector which contains the necessary elements for the transcription and translation of the inserted protein-coding sequence. Such transcription elements include a regulatory region and a promoter as defined previously. Thus, the nucleic acid encoding a marker compound of the invention is operably linked with a promoter in a expression vector, wherein said expression vector may include a replication origin.

The necessary transcriptional and translation of signals can be provided on an recombinant expression vector, or they may be supplied by the nucleic acid of the invention as defined above which is inserted in said vector.

### Structure of the vectors encompassed by the invention

A wide variety of host/expression vector combinations may be employed in expressing the nucleic acids of this invention. Useful expression vectors, for example, may consist of segments of chromosomal, non-chromosomal and synthetic DNA sequences. Suitable vectors include derivatives of SV40 and known bacterial plasmids, e.g., *Escherichia coli* plasmids col El, pCR1, pBR322, pMal-C2, pET, pGEX (Smith *et al*., 1988), pMB9 and their derivatives, plasmids such as RP4; phage DNAs, e.g., the numerous derivatives of phage I, e.g., NM989, and other phage DNA, e.g., M13 and filamentous single stranded phage DNA; yeast plasmids such as the 2m plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

For example, in a baculovirus expression systems, both non-fusion transfer vectors, such as but not limited to pVL941 (*Bam*H1 cloning site; Summers), pVL1393 (*Bam*H1, *Sma*I, *Xba*I, *Eco*R1, *Not*I, *Xma*III, *Bg/*II, and *Pst*I cloning site; Invitrogen), pVL1392 (*Bg/*II, *Pst*I, *Not*I, *Xma*III, *Eco*RI, *Xba*I, *Smal,* and *Bam*H1 cloning site; Summers and Invitrogen), and pBlue*Bac*III (*Bam*H1, *Bg/*II, *Pst*I, *Nco*I, and *Hind*III cloning site, with blue/white recombinant screening possible; Invitrogen), and fusion transfer vectors, such as but not limited to pAc700 (*Bam*H1 and *Kpn*I cloning site, in which the *Bam*H1 recognition site begins with the initiation codon; Summers), pAc701 and pAc702 (same as pAc700, with different reading frames), pAc360 (*Bam*H1 cloning site 36 base pairs downstream of a polyhedrin initiation codon; Invitrogen(195)), and pBlueBacHisA, B, C (three different reading frames, with *Bam*H1, *Bgl*II, *Pst*I, *Nco*I, and *Hind*III cloning site, an N-terminal peptide for ProBond purification, and blue/white recombinant screening of plaques; Invitrogen (220) can be used.

Mammalian expression vectors contemplated for use in the invention include vectors with inducible promoters, such as the dihydrofolate reductase (DHFR) promoter, e.g., any expression vector with *a DHFR* expression vector, or a *DHFR*/methotrexate co-amplification vector, such as pED (*Pst*I, *Sal*I, *Sba*I, *Sma*I, and *Eco*RI cloning site, with the vector expressing both the cloned gene and *DHFR;* Kaufman,1991). Alternatively, a glutamine synthetase/methionine sulfoximine co-amplification vector, such as pEE14 (*Hind*III, *Xba*I, *Smal, Sba*I, *Eco*RI, and *Bcl*I cloning site, in which the vector expresses glutamine synthase and the cloned gene; Celltech). In another embodiment, a vector that directs episomal expression under control of Epstein Barr Virus (EBV) can be used, such as pREP4 (*Bam*H1, *Sfi*I, *Xho*I, *Not*I, *Nhe*I, *Hind*III, *Nhe*I, *Pvu*II, and *Kpn*I cloning site, constitutive RSV-LTR promoter, hygromycin selectable marker; invitrogen), pCEP4 (*Bam*H1, *Sfi*I, *Xho*I, *Not*I, *Nhe*I, *Hind*III, *Nhe*I, *Pvu*II, and *Kpnl* cloning site, constitutive hCMV immediate early gene, hygromycin selectable marker; Invitrogen), pMEP4 (*Kpn*I, *Pvu*I, *Nhe*I, *Hind*III, *Not*I, *Xho*I, *Sfil, Bam*H1 cloning site, inducible methallothionein Ila gene promoter, hygromycin selectable marker: Invitrogen), pREP8 (*Bam*H1, *Xho*I, *Notl, Hind*III, *Nhe*I, and *Kpn*I cloning site, RSV-LTR promoter, histidinol selectable marker; Invitrogen), pREP9 (*Kpn*I, *Nhe*I, *Hind*III, *Not*I, *Xhol, Sti*I, and BamHI cloning site, RSV-LTR promoter, G418 selectable marker; Invitrogen), and pEBVHis (RSV-LTR promoter, hygromycin selectable marker, N-terminal peptide purifiable via ProBond resin and cleaved by enterokinase; Invitrogen). Selectable mammalian expression vectors for use in the invention include pRc/CMV (*Hind*III, *Bst*XI, *Not*I, *Sba*I, and *Apa*I cloning site, G418 selection; Invitrogen), pRc/RSV (*Hind*III, *Spe*I, *Bst*XI, *Not*I, Xbal cloning site, G418 selection; Invitrogen), and others. Vaccinia virus mammalian expression vectors (see, Kaufman, 1991, *supra)* for use according to the invention include but are not limited to pSC11 *(Smal* cloning site, TK- and b-gal selection), pMJ601 (*Sal*I, *Sma*I, *Afl*I, *Nar*I, *Bsp*MII, *Bam*HI, *Apa*I, Nhel, *Sac*II, *Kpnl,* and *Hind*III cloning site; TK- and b-gal selection), and pTKgptF1S (*Eco*RI, *Pst*I, *Sal*I, *Acc*I, *Hind*II, *Sba*I, *Bam*HI, and Hpa cloning site, TK or XPRT selection).

Yeast expression systems can also be used according to the invention to express a Selected Interacting Domain (SID®) polypeptide or a variant thereof and also a marker compound as defined herein. For example, the non-fusion pYES2 vector (*Xba*I, *Sph*I, *Sho*I, *Not*I, *Gst*XI, *Eco*RI, *Bst*XI, *Bam*H1, *Sac*I, *Kpn*1, and *Hind*III cloning sit; Invitrogen) or the fusion pYESHisA, B, C (*Xba*I, *Sph*I, *Sho*I, *Not*I, *Bst*XI, *Eco*RI, *Bam*H1, *Sac*I, *Kpn*I, and *Hindlll* cloning site, N-terminal peptide purified with ProBond resin and cleaved with enterokinase; Invitrogen), to mention just two, can be employed according to the invention.

Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e*.*g*., lambda), and plasmid and cosmid DNA vectors, to name but a few.

Vectors are introduced into the desired host cells by methods known in the art, e.g., transfection, electroporation, microinjection, transduction, cell fusion, DEAE dextran, calcium phosphate precipitation, lipofection (lysosome fusion), use of a gene gun, or a DNA vector transporter (see, *e.g.*, Wu et al., 1992; Wu and Wu, 1988; Canadian Patent Application No. 2,012,311, filed March 15, 1990).

A cell has been "transfected" by exogenous or heterologous DNA when such DNA has been introduced inside the cell. A cell has been "transformed" by exogenous or heterologous DNA when the transfected DNA effects a phenotypic change.

For introducing a vector in a cell host, explicit reference is made to research carried out by the group of E. Wagner, relating to gene delivery by means of plasmid-polylysine complexes (Curiel et al., 1992; and Curiel et al., 1992). The plasmid-polylysine complex investigated upon exposition to certain cell lines showed at least some expression of the gene. Further, it was found that the expression efficiency increased considerably due to the binding of transferrin to the plasmid-polylysine complex. Transferrin gives rise to close cell-complex contact with cells comprising transferrin receptors; it binds the entire complex to the transferrin receptor of cells. Subsequently, at least part of the entire complex was found to be incorporated in the cells investigated.

Several different approaches have been developed for gene transfer. These include the use of viral based vectors (e.g., retroviruses, adenoviruses, and adeno-associated viruses) (Drumm, M. L. et al., Rosenfeld, M. A. et al., 1992; and Muzyczka, 1992), charge associating the DNA with an asialorosomucoid/poly L-lysine complex (Wilson, J. M. et al. 1992), charge associating the DNA with cationic liposomes (Brigham, K. L. et al., 1993) and the use of cationic liposomes in association with a poly-L-lysine antibody complex (Trubetskoy, V. S. et al., 1993).

### Compositions comprising vectors of the invention.

Although non-viral based transfection systems have not exhibited the efficiency of viral vectors, they have received significant attention, in both in vitro and in vivo research, because of their theoretical safety when compared to viral vectors. Synthetic cationic molecules, have been reported which reportedly "coat" the nucleic acid through the interaction of the cationic sites on the transfection agent and the anionic sites on the nucleic acid. The positively charged coating reportedly interacts with the negatively charged cell membrane to facilitate the passage of the nucleic acid through the cell membrane by non-specific endocytosis. (Schofield, 1995) These compounds have, however, exhibited considerable sensitivity to natural serum inhibition, which has probably limited their efficiency in vivo as gene transfection agents. (Behr1994)

A number of attempts have been made to improve the efficiency of lipid-like cationic transfection agents, some involving the use of polycationic molecules. For example, several transfection agents have been developed that contain the polycationic compound spermine covalently attached to a lipid carrier. (Behr, 1994), discloses a lipopolyamine and shows it to be more efficient at transfecting cells than single charge molecules (albeit still less efficient than viral vectors). The agent reported by Behr was, however, toxic, and caused cell death.

A few such lipid delivery systems for transporting DNA, proteins, and other chemical materials across membrane boundaries have been synthesized by research groups and business entities. Most of the synthesis schemes are relatively complex and generate lipid based delivery systems having only limited transfection abilities. A need exists in the field of gene therapy for cationic lipid species that have a high biopolymer transport efficiency. It has been known for some time that a very limited number of certain quaternary ammonium derivatized (cationic) liposomes spontaneously associate with DNA, fuse with cell membranes, and deliver the DNA into the cytoplasm (as noted above, these species have been termed "cytofectins"). LIPOFECTIN TM. represents a first generation of cationic liposome formulation development. LIPOFECTIN TM is composed of a 1:1 formulation of the quaternary ammonium containing compound DOTMA and dioleoylphosphatidylethanolamine sonicated into small unilamellar vesicles in water. Problems associated with LIPOCFECTIN TM include non-metabolizable ether bonds, inhibition of protein kinase C activity, and direct cytotoxicity. In response to these problems, a number of other related compounds have been developed. The monoammonium compounds of the subject invention improve upon the capabilities of existing cationic liposomes and serve as a very efficient delivery system for biologically active chemicals.

Since the original report (Feigner et al.), that liposomes comprised of equal amounts of the cytofectin DOTMA (N->1-(2,3-dioleyloxy)propyl!-N,N,N-trimethylammonium chloride) and neutral lipid DOPE (dioleoyl phosphotidylethanolamine) spontaneously associate with DNA to form efficient transfection complexes, the technology has advanced incrementally. There have been few cytofectins developed which have improved upon the *in vivo* activity of the prototypic agent DOTMA. This lack of progress may reflect funding priorities which have focused on the application of cationic lipid technology to biologic problems, rather than research focusing on principles which effect cytofectin-mediated gene delivery. Specifically, studies focused on the mechanism(s) involved in cytofectin actions, barriers to cytofectin-mediated *in vivo* gene delivery, and clarification of cytofectin structure/activity relationships would facilitate the development of improved cationic lipid-based delivery reagents. While research into the mechanism responsible for cationic amphiphile-mediated gene delivery is ongoing in a number of laboratories (Sternberg et al., Wrobel et al.,1995 and Zabner et al. 1995), even the most basic aspects of the mechanism of action of cytofectins (the relative contributions of direct cytoplasmic membrane fusion and endocytosis) remain unresolved.

As indicated above, various cationic lipids have been synthesized in previous references. In the realm of patents, for example, U.S. Pat. No. 4,812,449 discloses in situ active compound assembly of biologically active agents at target locations in preference to surroundings which are desired to be unaffected. Several charged and uncharged amine derivatives are described. Introduced in U.S. Pat. No. 5,171,678 are lipopolyamines and their use for transfecting eukaryotic cells. A polynucleotide is mixed with the subject lipopolyamine and contacted with the cells to be treated. U.S. Pat. Nos. 5,186,923 and 5,277,897 relate an enhancement of cellular accumulation of lipophilic cationic organometallic compounds by reduction of the intramembrane potential. Technetium containing compounds are disclosed. Lipophilic cationic compounds are presented in U.S. Pat. No. 5,208,036. Asymmetrical amine compounds are synthesized and employed in a method for DNA transfection. The amines are quaternized by two hydrogens or alkyl, aryl, aralkyl, quinuclidino, piperidino, pyrrolidino, or morpholine groups, unlike the present invention.

U.S. Patent No. 5,264,618 discloses cationic lipids for intracellular delivery of biologically active molecules. Asymmetric ammonium containing cationic lipids are presented for transporting molecules into membrane enclosed systems. The amines are quaternized by two hydrogens or alkyl groups, unlike the present invention. Transfection of nucleic acids into animal cells via a neutral lipid and a cationic lipid is revealed in U.S. Pat. No. 5,279,833. Liposomes with nucleic acid transfection activity are formed from the neutral lipid and the ammonium salt containing cationic lipid.

U.S. Patent No. 5,334,761 describes other amine containing cationic lipids. Cationic lipids are utilized to form aggregates for delivery of macromolecules and other compounds into cells. The amines are quaternized by two hydrogens or unbranched alkyl groups, unlike the present invention.

In the PCT publication of PCT/US 94/13362 a heterocyclic diamine is disclosed. A symmetrical quaternary diamine having lipid tails is related for forming liposomes.

Another method for achieving delivery of DNA containing liposomes involves the administration of complexes of DNA with cationic lipids. For example, U.S. Pat. No. 5,227,170 teaches encapsulation of oligonucleotides in cationic lipid complexes which further comprise a divalent cation solution containing the desired oligonucleotides and which have an osmolarity less than that of the internal aqueous phase (Felgner et al., 1987). Due to their cationic character, these liposomes can bind to serum proteins, thereby leading to inactivation of the oligonucleotides contained therein. Also, osmotic-dependent liposomes are known in the literature, i.e., as disclosed by U.S. Pat. No. 5,049,392.

Methods for optimizing the size of liposomes also are known. For example, U.S. Pat. No. 4,532,089 teaches a method for preparing giant liposomes. Also, U.S. Pat. No. 4,529,561 teaches a method for preparing liposomes in desired size ranges. Further, U.S. Pat. No. 5,223,263 teaches liponucleotide containing liposomes and the use thereof in delivery of the liponucleotides to desired cells.

### Most preferred vectors of the invention.

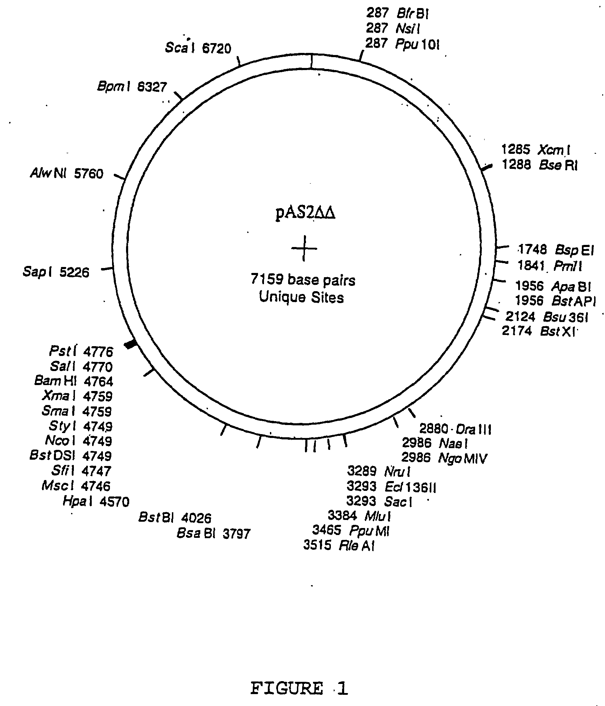

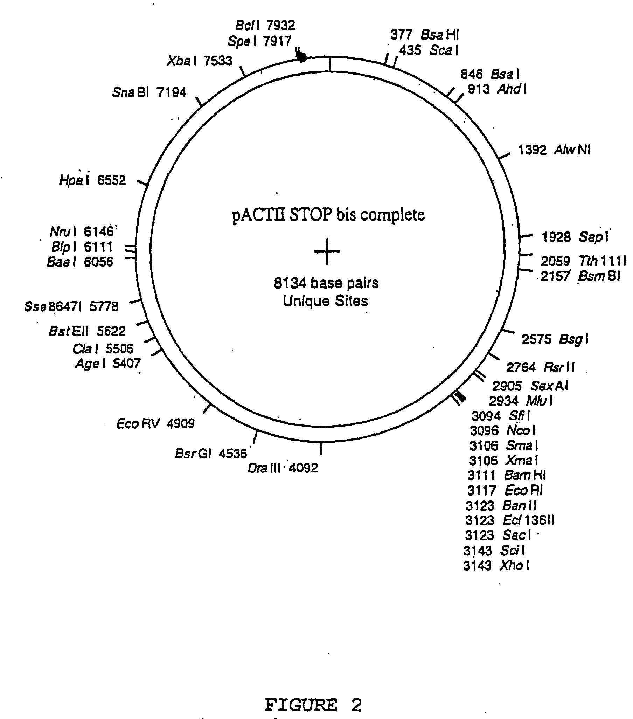

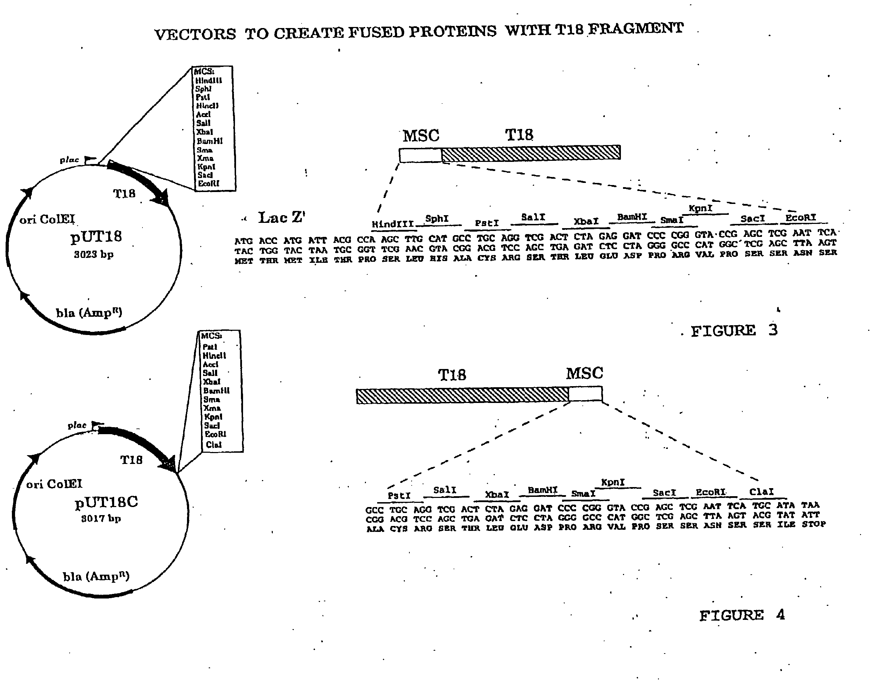

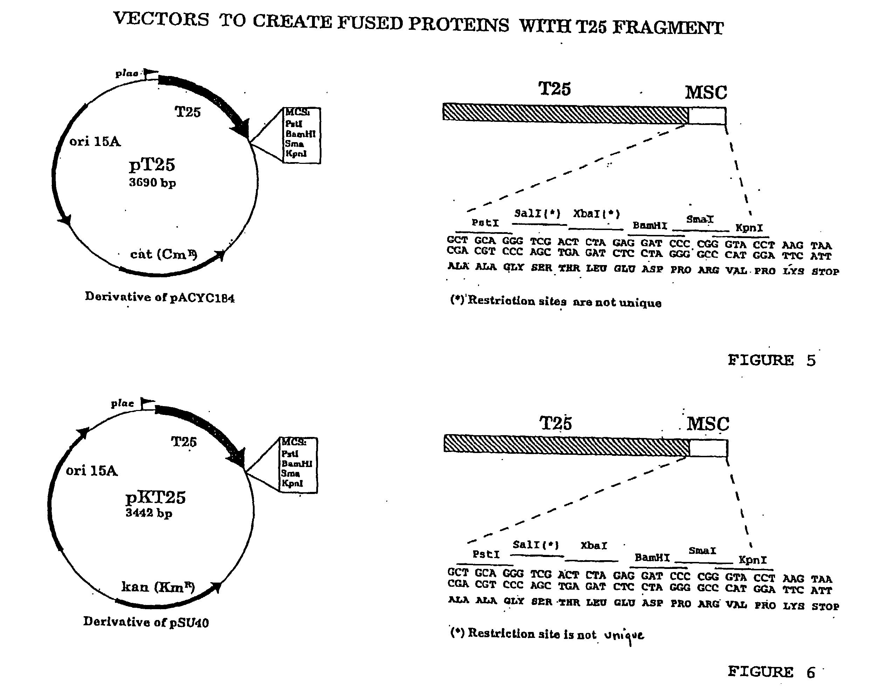

Most preferred recombinant vectors according to the invention include pASΔΔ(figure 1), pACTllst (figure 2), pT18(figure 3), pUT18C (figure 4), pT25 (figure 5) and pKT25(figure 6) containing inserted therein a nucleic acid encoding a Selected Interacting Domain (SID®) polypeptide or a variant thereof or encoding a marker compound of the invention.

In the specific embodiment wherein the nucleic acid inserted in a vector above encodes for a fusion protein, such as a fusion protein between, a Selected Interacting Domain (SID®) polypeptide and a fluorescent protein (e.g. GFP or YFP) or an enzyme or a catalytically active fragment thereof (e.g. alkaline phosphatase, glutathion peroxydase, horse radish peroxydase or β-galactosidase), the one skilled in the art may advantageously refer to the general literature, for example bibliographic references pertaining to the production of fusion proteins between an antibody or an antibody fragment and a protein label. Fusion proteins have been widely used in immunoassays. Preferably, the one skilled in the art may refer to the article of Peterhans et al. (1987), to the US Patent N°4,745,055, to the article of Handl et al. (1988), to the US Patent N°4,774,175.

For designing a recombinant vector encoding a marker compound of the invention wherein the detectable molecule consist of a green fluorescent protein (GFP), the one skilled in the art may also refer to the article of Adams et al. (1995).

In the embodiment according to which the nucleic acid inserted in a vector of the invention encodes for a Selected Interacting Domain (SID®) polypeptide fused to a green fluorescent protein (GFP), the one skilled in the art may design such a nucleic acid starting:
- on the one hand from the nucleic acid encoding the Selected Interacting Domain (SID®) polypeptide under consideration; and
- on the other hand from a nucleic acid encoding a GFP protein, which may be the nucleic acid inserted in the vector lambda. GFP10, such as described by Prascher et al. (1992).

### RECOMBINANT HOST CELLS

In one embodiment, Selected Interacting Domain (SID®) polypeptide or a variant or also a marker compound of the invention is recombinantly produced in a desired host cell which has been transfected or transformed with a nucleic acid encoding said marker compound or with a recombinant vector as defined above within which a nucleic acid encoding a marker compound of the invention is inserted.

Recombinant host cells are another aspect of the present invention.

Such host cells generally comprise at least one copy of a nucleic acid encoding a Selected Interacting Domain (SID®) polypeptide or a variant thereof or encoding a marker compound of the invention.

Preferred cells for expression purposes will be selected in function of the objective which is sought. For example, in the embodiment wherein the production of a marker compound according to the invention in large quantities is sought, the nature of the cell host used for its production is relatively indifferent, provided that large amounts of Selected Interacting Domain (SID®) polypeptides or marker compounds of the invention are produced and that optional further purification steps may be carried out easily.

However, in the embodiment wherein the marker compound is recombinantly produced within a host organism for the purpose of qualitative or quantitative analysis of the polypeptide of interest onto which said marker compound specifically binds, then the host organism is selected among the host organisms which are suspected to produce naturally said polypeptide of interest.

Consequently, mammalian and human cells, as well as bacterial, yeast, fungal, insect, nematode and plant cells are cell host encompassed by the invention and which may be transfected either by a nucleic acid or a recombinant vector as defined above.

Examples of suitable recombinant host cells include VERO cells, HELA cells (e.g. ATCC N°CCL2), CHO cell-lines (e.g. ATCC N°CCL61) COS cells (e.g. COS-7 cells; COS cell referred to ATCC N°CRL1650), W138, BHK, HepG2, 3T3 (e.g. ATCC N°CRL6361), A549, PC12, K562 cells, 293 cells, Sf9 cells (e.g. ATCC N°CRL1711) and Cv1 cells (e.g. ATCC N°CCL70).

Other suitable host cells are usable according to the invention include prokaryotic host cells strains of *Escherichia coli* (e.g. strain DH5-α), of *Bacillus subtilis,* of *Salmonella typhimurium*, or strains of genera such as *Pseudomonas*, *Streptomyces* and *Staphylococcus*.

Further suitable host cells usable according to the invention include yeast cells such as those of *Saccharomyces*, typically *Saccharomyces cerevisiae.*

### DETECTION METHODS OF THE INVENTION

The present invention further relates to the use of a Selected Interacting (SID®) polypeptide or a variant thereof as well as a nucleic acid encoding it for detection purposes. It is herein reminded that a Selected Interacting Domain (SID®) polypeptide is determined according to the ability of such a (SID®) polypeptide to bind in a highly specific manner to a given (e.g. bait) polypeptide of interest, since the aminoacid sequence of a SID® polypeptide is encoded by a nucleic acid, the nucleotide sequence of which consists of the polynucleotide sequence which is common to a collection of nucleic acid sequences encoding prey polypeptides that have been selected for their specific binding properties to a (bait) polypeptide of interest, such as explained above in the section entitled « SELECTED INTERACTING DOMAIN (SID®) POLYPEPTIDES ».

The specific properties of a Selected Interacting Domain (SID®) polypeptide for binding to a given polypeptide of interest, either a viral, yeast, fungal, bacterial, insect, plant or mammal polypeptide, including a polypeptide of human origin, allow its use as a specific ligand for said polypeptide of interest of which the detection is sought.

Therefore, the use of a Selected Interacting Domain (SID®) in any detection method known in the art and which makes use of the ability of a detection ligand to bind specifically to a molecule of interest, most preferably a polypeptide of interest, fall under the scope of the present invention.

Detection methods that make use of the recognition of a molecule of interest, most preferably a polypeptide of interest, by a detection ligand are well known in the art and are primarily illustrated by the abundant literature that relate to immunoassays, which is incorporated herein by reference in its entirety.

The one skilled in the art may particularly refer to the book of Maggio (1980) (Heterogeneous assays), the US Patent N°3,817,837 (homogeneous Immunoassays), US Patent N° 3,993,345 (Immunofluorescense methods), US Patent N°4,233,402 (enzyme channelling techniques), US Patent N°3,817,837 (Enzyme multiplied immunoassay technique), US Patent N°4,366,241 and European Patent Application N°EP-A 0 143 574 (Migration type assays), US Patent N°5,202,006, US Patent N°5,120,413 and US Patent N°5,145,567 (Immunofixation electrophoresis, mmunoelectrophoresis), the article of Aguzzi et al. (1977), the article of White et al. (1986), the article of Merlini et al. (1983), the US Patent n°5,228,960 (Immunosubstraction electrophoresis), the articles of Chen et al. (1991), Nielsen et al. (1991) and the US Patent n° 5,120,413 (Capillary electrophoresis).

### Acellular detection method of the invention.

A first detection method of the invention consists of a method for detecting a polypeptide of interest within a sample, wherein said method comprises the steps of:
a) contacting a marker compound or a plurality of marker compounds according to the invention with the sample which is suspected to contain the polypeptide of interest the detection of which is sought;
b) detecting the complexes formed between said marker compound or said plurality of marker compounds and said polypeptide of interest.

The sample which is assayed for the presence of the polypeptide of interest the detection of which is sought may be of any nature , including every sample that may be used for carrying out an immunoassay.

In a first aspect, the sample may be any biological fluid, such as blood or blood separation products (e.g. serum, plasma, buffy coat), urine, saliva, tears.

In a second aspect, the sample may be any isolated biological tissue sample, including tissue sections previously fixed for purposes of histological studies.

In a third aspect, the sample may be a culture supernatant of a cell culture and a cell lysate of cultured cells.

In a first preferred embodiment of the first detection method of the invention described above, the detection step b) consists of the measure of the fluorescence signal intrinsically emitted by the detectable molecule. It may for exampole be taken the advantage of SID® polypeptides or variants thereof having in their aminoacid sequence one or several tryptophan aminoacid residues.

In a second preferred embodiment of the first detection method of the invention detailed above, the detection step b) consists of submitting the detectable molecule to a source of energy at the excitation wavelength of said detectable molecule, and measuring the light emitted at the emission wavelength of said detectable molecule.

An illustrative example of this second embodiment above is when the marker compound used consists of a Selected Interacting Domain (SID®) which is bound to a fluorescent molecule, such as fluorescent protein like GFP and YFP.

For example, in the embodiment wherein the detectable molecule of the marker compound of the invention which is used according to the first detection method above comprises, or alternatively consists of, a GFP protein, the detection step c) includes illuminating the sample tested at an emission wavelength substantially equal to 490 nm, and measuring the light emitted by the marker compound which is bound to the polypeptide of interest within the sample at an emission wavelength substantially equal to 510 nm.

Preferably, the marker compounds which are not bound to the polypeptide of interest the detection of which is sought within the sample are removed before carrying out the detection step.

In a third preferred embodiment, the detection step c) of the first detection method of the invention consists of measuring the catalytic activity of the detectable molecule. In this specific embodiment, it flows that the marker compound used in the detection method comprises a detectable molecule which comprises, or alternatively which consists of, an enzyme or a catalytically active enzyme fragment, such as already detailed in the section entitled « Marker compounds of the invention ».

In a fourth preferred embodiment, the detection step b) consists of measuring the radioactivity emitted by the detectable molecule.

The present invention further relates to a kit for detecting a polypeptide of interest within a sample, wherein said kit comprises a marker compound according to the invention.

Optionally, said detection kit further comprises the reagents necessary for carrying out the detection step b), such as a suitable substrate for the particular enzyme or a catalytically active enzyme fragment used, as well as suitable buffer solutions, which may be identical to those conventionally used for performing immunoassays.

### Cellular detection assay using a recombinantly produced marker compound of the invention.

As already described above, any marker compound according to the invention may be produced according to genetic engineering techniques. Particularly, nucleic acid encoding a particular marker compound which binds specifically to a polypeptide of interest the detection of which is sought may be inserted in a vector, wherein said vector may be used to transfect or transform a host organism, either a prokaryotic or an eukaryotic cell host such as defined above.

In this specific embodiment, the production of a recombinant marker compound of the invention is allowed within such a transfected or transformed host cell. Once the host cell of interest is transfected or transformed with such a recombinant vector and once the recombinant marker compound is produced within the cell host of interest, then the Selected Interacting Domain (SID®) polypeptide portion of said marker compound will be able to bind specifically with its specific target polypeptide within the cell host. In this situation, the recombinantly produced marker compound of the invention will predominantly be localised at cell sites wherein the targeted polypeptide of interest is present.

This is the purpose of the second detection method of the invention which is detailed below.

A further object of the invention consists of a method for detecting a polypeptide of interest within a prokaryotic or an eukaryotic cell host, wherein said method comprises the steps of:
a) providing a cell host to be assayed;
b) transfecting said cell host with a nucleic acid encoding a marker compound of the invention, or with a recombinant vector encoding a marker compound of the invention;
c) detecting the complexes formed between the marker compound expressed by the transfected cell host and the polypeptide of interest.

Because the Selected Interacting Domain (SID®) polypeptide which is part of a marker compound of the invention specifically binds to a polypeptide which is suspected to be naturally produced by the targeted cell host, the second detection method of the invention defined above allows a qualitative as well as a quantitative detection of this targeted polypeptide which is suspected to be naturally produced by the transfected target cell host under assay.

For example, in the embodiment within which the procedure for selecting the Selected Interacting Domain (SID®) polypeptide which is part of a marker compound of the invention includes a first step wherein a collection of clones containing nucleic acid inserts derived from a viral genomic DNA library is prepared, the transfection of a mammalian cell, preferably a human cell, with a vector encoding such a marker compound of the invention will allow to detect the expression of a human polypeptide naturally expressed within said mammalian host cell and which naturally interacts with the viral protein from which is derived the Selected Interacting Domain (SID®) polypeptide.

In another embodiment, the Selected Interacting Domain (SID®) polypeptide which is part of a marker compound of the invention might have been selected according to the above described procedure, the first step of which was performed with clones containing nucleic acid inserts deriving from a genomic DNA or cDNA human library. In this embodiment, the expression of such a marker compound in a human cell previously transfected with the vector having the ability to express this marker compound in the human cell host will allow the detection of a targeted polypeptide naturally expressed within said human cell host and which naturally interacts with the human protein from which is derived the Selected Interaction Domain (SID®) polypeptide.

The second detection method of the invention defined above firstly allows the qualitative detection of the targeted polypeptide of interest which binds specifically with the recombinantly produced marker compound of the invention, and thus permits to know in which environmental conditions or at which differentiation stage the targeted polypeptide of interest is naturally produced within the cell host transfected with a vector expressing a marker compound of the invention.

Secondly, this second detection method of the invention allows the localisation of the targeted polypeptide of interest within the interior of the cell, including localisation in plasma membrane, cytosol, nucleus and any organelle such as ribosomes, Golgi apparatus, lysosomes, phagosomes, endoplasmic reticulum and chloroplasts.

The localisation of a targeted polypeptide of interest which is expressed within the cell host under assay according to the second detection method of the invention may be carried out by any means well known in the art, including using a confocal microscope.

Thirdly, the second detection method of the invention allows also a quantitative analysis of the expression of the targeted polypeptide of interest within the cell host under assay, since the level of the detection signal produced by the detectable molecule which is part of the marker compound will be proportional to the number of complexes formed between the cell host under assay between the targeted polypeptide of interest and the recombinantly produced marker compound of the invention.

Essentially, the one skilled in the art may refer to the section entitled « A cellular detection method of the invention » above to find the teachings necessary for performing the detection step c) of the second detection method described herein.

In a first embodiment of said second detection method of the invention, the detection step c) consists of the measure of the fluorescence signal intrinsically emitted by the detectable molecule comprised in the recombinantly expressed marker compound of the invention.

In a second preferred embodiment of the second detection method above, the detection step c) consists of submitting the detectable molecule to a source of energy at the excitation wavelength of said detectable molecule and measuring the light emitted at the emission wavelength of said detectable molecule.

In still a further embodiment of the second detection method of the invention, the detection step c) consists of measuring the catalytic activity of the detectable molecule.

in another embodiment, the detection step c) consists of measuring the radioactivity emitted by the detectable molecule.

In yet a further embodiment of the second detection method of the invention, the detection step c) allows the location of the complexes formed between the recombinantly produced marker compound and the targeted polypeptide of interest within the transfected cell host.

A further object of the invention consists of a kit for detecting a polypeptide of interest within a prokaryotic or an eukaryotic cell host, wherein said kit comprises a nucleic acid encoding a marker compound as defined herein, or a recombinant vector containing inserted therein a nucleic acid encoding a marker compound of the invention.

Optionally, the detection kit above may further comprise the reagents necessary to carry out the detection step c).

### Cellular detection method of the invention using a marker compound which is introduced within a cell host.

There is a third detection method according to the invention wherein the marker compound comprising a Selected Interacting Domain (SID®) polypeptide or a variant thereof is previously produced by any means and subsequently introduced into a target cell host for the purpose of detecting a targeted polypeptide of interest which binds specifically with said Selected Interacting Domain (SID®) polypeptide.

Thus, the invention further relates to a method for detecting a polypeptide of interest within a prokaryotic or an eukaryotic cell host, wherein said method comprises the step of:
a) providing a cell host to be assayed;
b) introducing a marker compound as defined herein within said cell host; and
c) detecting the complexes formed between the marker compound and the polypeptide of interest within the cell host.

Taking into account the low molecular weight of the Selected Interacting Domain (SID®) polypeptide which is part of a marker compound of the invention, when compared with conventional specific detection molecules such as antibodies or antibody fragments, it flows that the introduction of a marker compound of the invention into the interior of a target cell host will be much more easier to perform, as compared with the introduction within a cell host of a conventional marker like a labelled antibody or a labelled antibody fragment.

According to the third detection method of the invention defined above, step b) of introducing the marker compound within the target cell host may be performed by any technique well known in the art, including electroporation, and the use of molecules that will facilitate the passage of the marker compound of the invention through the cell membranes, and typically the plasma membrane.

Such molecules that facilitate the passage of a marker compound of the invention through cell membranes include, but are not limited to, penetratin, like penetratin 1.RTM (Encor, Gaithersburg, Md), Antenna Pediae protein, cationic lipids and cationic polyacrylates.

Permeation enhancers which may be employed include bile salts such as sodium glycocholate and other molecules such as β-cyclodextrin. Bile salts are known to increase the absorption of macromolecules across membranes (Pontiroli et al., 1987).

As already detailed for the second detection method of the invention described in the previous section, the third detection method of the invention allows also the localisation of the targeted polypeptide of interest which is expressed by the cell host under assay, as well as the qualitative and quantitative analysis of the expression of said target polypeptide of interest.

The detection step c) according to the third detection method of the invention described above may be carried out in the same way than the detection step c) of anyone of the first detection method and the second detection method detailed in the previous sections herein.

In a first embodiment of the third detection method above, the detection step c) consists of the measure of the fluorescence signal intrinsically emitted by the detectable molecule.

In a second embodiment, the detection step c) consists of submitting the detectable molecule to a source of energy at the excitation wavelength of said detectable molecule and measuring the light emitted at the emission wavelength of said detectable molecule.

In a third embodiment, the detection step c) consists of measuring the catalytic activity of the detectable molecule.

In a fourth embodiment, the detection step c) consists of measuring the radioactivity emitted by the detectable molecule.

In a fifth embodiment of the third detection method of the invention, the detection step c) allows the location of the complexes formed between the marker compound and the polypeptide of interest within the target cell host under assay.

A further object of the invention consists of a kit for detecting a polypeptide of interest within a prokaryotic or an eukaryotic cell host, wherein said kit comprises a marker compound as defined herein.

The detection kit above may further comprise the reagents necessary to carry out the detection step c).

The detection kit above may also further comprise the reagents necessary to facilitate the introduction of the marker compound within the target cell host under assay.

### SOLID PHASE DETECTION METHOD USING A SELECTED INTERACTING DOMAIN (SID®) POLYPEPTIDE.

In a further aspect of the invention, the use of a Selected Interacting Domain (SID®) polypeptide or a variant thereof for detection purpose include a step wherein said Selected Interacting Domain (SID®) polypeptide is immobilised on a suitable substrate before bringing a sample to be assayed in contact with the substrate onto which said Selected Interacting Domain (SID®) polypeptide has been previously immobilised.

A subsequent step will consist in detecting the complexes formed between the Selected Interacting Domain (SID®) polypeptide immobilised on the substrate and the targeted polypeptide of interest the presence of which is suspected in the sample assayed.

Thus, the invention also pertains to a fourth detection method which consists of a method for detecting a polypeptide or a plurality of polypeptides of interest within a sample, wherein said method comprises the steps of :
a) providing a substrate onto which a Selected Interacting Domain (SID®) polypeptide or a plurality of Selected Interacting Domain (SID®) polypeptides is (are) immobilised;
b) bringing into contact the substrate defined in a) with the sample to be assayed;
c) detecting the complexes formed between the Selected Interacting Domain (SID®) polypeptide or the plurality of Selected Interacting Domain (SID®) polypeptides and the target polypeptide or the plurality of target polypeptides contained in the sample.

Substrates, supports or surfaces for immobilising protein molecules are well known in the art, and a lot of them have been described for performing solid phase immunoassays.

Preferably, a plurality of Selected Interacting Domain (SID®) polypeptides of different aminoacid sequences are immobilised on the substrate used according to the fourth detection method of the invention.

For example, a complete collection of Selected Interacting Domain (SID®) polypeptides which have been determined according to the methods described in the section entitled « Selected Interacting Domain (SID®) polypeptides » above, using nucleic acids derived from a given cell host genomic DNA or cDNA as starting material, may be used for being immobilised on a suitable substrate.

According to this embodiment, the collection of Selected Interacting Domain (SID®) polypeptides are immobilised on the substrate in another manner, thus forming an ordered area of SID® polypeptides immobilised at known locations of the surface of said substrate.

The substrate, support or surface may be a porous or a nonporous water insoluble material. The support can be hydrophilic or capable of being rendered hydrophilic and includes inorganic powders such as silica, magnesium sulphate, and alumina; natural polymeric materials, particularly cellulosic materials and materials derived from cellulose, such as fiber containing papers; synthetic or modified naturally occurring polymers, such as nitro-cellulose, cellulose acetate, poly(vinyl chloride), polyacrylamide , cross-linked dextran, agarose, polyacrylate, polyethylene, polypropylene, poly(4-methylbutene), polystyrene, polymethacrylate, poly(ethylene terephtalate), nylon, poly(vinyl butyrate), said materials being used by themselves or in conjunction with other materials; glass available as Bioglass, ceramic metals and the like.

Binding of a Selected Interacting Domain (SID®) polypeptide or of a plurality of Selected Interacting Domain (SID®) polypeptide to the substrate, support or surface may be accomplished by wellknown techniques, commonly available in the literature. The substrate, surface or support can have anyone of a number of shapes, including strip, rod or particles like beads.

The surface of the substrate or support will usually be polyfunctional or be capable of being polyfonctionalized or be capable of binding a Selected Interacting Domain (SID®) polypeptide through specific or non-specific covalent or non-covalent interactions. A wide variety of functional groups for linking are available in the art. The length of a linking group may vary widely, depending notably upon the effect of the distance between the SID® polypeptide being linked and the support on the assay.

As illustrative examples, the one skilled in the art may advantageously refer to the US Patent N°4,168,146 (« Immunoassay with test strip having antibodies bound thereto »), and to the US Patent N°4,347,311 (« enzyme immunoassay for determining antigens specific antibodies and test kit for carrying out this assay »).

An ordered area onto which a plurality of Selected Interacting Domain (SID®) polypeptides are immobilised may be manufactured according to the techniques disclosed in the US Patent N°5,143,854 or the PCT Application n°WO 92/10092, incorporated herein by reference for all purposes. The combination of photolithographic and fabrication techniques may, for example, enable each Selected Interacting Domain (SID®) polypeptide to occupy a very small area (« site ») on the support. In some embodiments, the site may be as small as few microns or even a single Selected Interacting Domain (SID®) polypeptide.

For example, about 10⁵ to 10⁶ Selected Interacting Domain (SID®) different polypeptides may be immobilized in an area of only 12.8 mm². Such probe areas may be of the type known as Very Large Scale Immobilized polymer synthesis, which is a brand name belonging to Affymetrix.

In a first embodiment of the fourth detection method detailed above, the plurality of Selected Interacting Domain (SID®) polypeptides are immobilized on the substrate in an order manner.

In a second embodiment of Selected Interacting Domain (SID®), the Selected Interacting Domain (SID®) polypeptide or the plurality of Selected Interacting Domain (SID®) polypeptides are covalently bound to the substrate.

In a third embodiment of said method, the Selected Interacting Domain (SID®) polypeptide or the plurality of Selected Interacting Domain (SID®) polypeptides are non-covalently bound to the substrate. According to this specific embodiment, the Selected Interacting Domain (SID®) polypeptide or the plurality of Selected Interacting Domain (SID®) polypeptides are covalently bound to a first ligand molecule and the substrate is coated with a second ligands molecule, wherein said second ligand molecule specifically binds to the first ligand molecule. According to such a specific embodiment, the first ligand may be biotin in which case the second ligand is most preferably streptavidin.

In still a further embodiment of the fourth detection method according to the invention, the Selected Interacting Domain (SID®) polypeptide or the plurality of Selected Interacting Domain (SID®) polypeptides are covalently linked to a spacer, which spacer is itself also covalently bound to the substrate in order to immobilise the Selected Interacting Domain (SID®) polypeptide or the plurality of Selected Interacting Domain (SID®) polypeptides onto said substrate. Such a spacer may be a peptide polymer such as a poly-alanine or a poly-lysine peptide of 10 to 15 amino acids in length.

In still a further embodiment of the fourth detection method above, the detection step c) consists of detecting changes in the optical characteristics of the substrate onto which the Selected Interacting Domain (SID®) polypeptide or the plurality of Selected Interacting Domain (SID®) polypeptides are bound.

In yet a further embodiment of the fourth detection method of the invention, the detection step c) consists of bringing into contact the substrate wherein complexes are formed between the targeted polypeptide molecule contained in the sample assayed and the Selected Interacting Domain (SID®) polypeptide or the plurality of Selected Interacting Domain (SID®) polypeptides bound to said support, with a detectable molecule having the ability to bind to such complexes.

According to the specific embodiment, the detectable molecule may be any of the detectable molecules that are part of a marker compound of the invention that are defined in the section entitled « marker compounds of the invention » above and which are able to recognize the complexes formed between the immobilised Selected Interaction Domain polypeptide and the polypeptides of the sample under assay.

According to this specific embodiment, the detectable molecule may also consists of a Selected Interacting Domain (SID®) polypeptide or a plurality of Selected Interacting Domain (SID®) polypeptides which have been radioactively or non-radioactively labelled.

In the embodiment wherein the detectable molecule used for performing the fourth detection method of the invention is fluorescent, the detection step c) may be carried out with any device for detecting fluorescent marker targets, including systems comprising a microscope and a monochromatic or polychromatic light source for directing light at a substrate.

A photo counter detects fluorescence from the substrate, while an x-y translation stage varies the location of the substrate.

A computer controls the movement of the x-y translation table and data collection. Such suitable devices are for example those described in the US Patent N°5,143,854 and PCT Application N°WO 92/10092.

Another suitable device is disclosed in the US Patent N°5,631,734.

Other suitable devices include scanning microscopes that employ radiation direction systems, such as galvanometers that include servo-mounted mirrors, to rapidly scan a laser spot across the substrate, such as those disclosed in US Patent N°5,143,854 or in the US Patent N°5,981,956.

The Biacore ™ technology can also be used to carry out the screening of compounds capable of interacting with said one of the set of two polypeptides of the invention. This technology is described in Szabo et al. (1995) and in Edwards and Leartherbarrow (Analytical Biochemistry, 246, 1-6, 1997), of which the teaching is incorporated by reference, and makes it possible to detect interactions between molecules in real time without the use of labelling.

A further object of the invention consists of a device or an apparatus for the detection of a polypeptide or a plurality of polypeptides of interest within a sample, wherein said device or apparatus comprises a substrate onto which a Selected Interacting Domain (SID®) polypeptide or a plurality of Selected Interacting Domain (SID®) polypeptide is (are) immobilised.

Such a device or apparatus of the invention above may comprise or consist of a suitable substrate onto which the plurality of Selected Interacting Domain (SID®) polypeptides are arranged in an ordered manner, thus forming an area such as described above.

### A METHOD FOR LABELLING A POLYPEPTIDE OF INTEREST USING A MARKER COMPOUND OF THE INVENTION.

As it is already disclosed hereinbefore, a Selected Interacting Domain (SID®) polypeptide or a variant thereof, is determined according to the ability of such a polypeptide to bind in a highly specific manner to a given polypeptide of interest.

Consequently, a Selected Interacting Domain (SID®) polypeptide or a variant thereof may be used for labelling a given polypeptide of interest onto which said Selected Interacting Domain (SID®) polypeptide or said variant thereof binds specifically.

Thus, the present invention also concerns a method for labelling a polypeptide of interest, wherein said method comprises the steps of:
a) provided a Selected Interacting Domain (SID®) polypeptide which binds specifically to said polypeptide of interest;
b) bringing into contact the Selected Interacting Domain (SID®) polypeptide with said polypeptide of interest, whereby said polypeptide of interest will be labelled.

The labelling of a polypeptide of interest may also be performed by using a marker compound as defined herein, which comprises a Selected Interacting Domain (SID®) polypeptide which binds specifically to the polypeptide of interest the labelling of which is sought.

Consequently, the invention further relates to a method for labelling a polypeptide of interest, wherein said method comprises the steps of:
a) providing a marker compound of the invention which binds specifically to said polypeptide of interest;
b) bringing into contact said marker compound with said polypeptide of interest, whereby said polypeptide of interest will be labelled.

The one skilled in the art will be able to determine the suitable conditions to be carried out for performing a polypeptide labelling method described above by referring to the abundantly literature?? directed to the labelling of proteins with antibodies.

For specific labelling techniques, the one skilled in the art may also refer to the numerous bibliographic references which are referred to within the present specification under the section entitled « Marker compounds of the invention ».

Another object of the invention consists of a kit for labelling a polypeptide of interest wherein said kit comprises a Selected Interacting Domain (SID®) polypeptide and optionally the reagents necessary for performing the labelling procedure.

A further object of the invention consists of a kit for labelling a polypeptide of interest, wherein said kit comprises a marker compound according to the invention and optionally the reagents necessary for performing the labelling procedure.

### PURIFICATION OF A POLYPEPTIDE OF INTEREST USING A SELECTED INTERACTING DOMAIN (SID®) POLYPEPTIDE.

Taking advantage of the highly specific binding properties of a Selected Interacting Domain (SID®) polypeptide or a variant thereof to a given polypeptide of interest, any Selected Interacting Domain (SID®) polypeptide which is specific for a given polypeptide of interest may be used in purification methods of said polypeptide of interest.

For such a purpose, a Selected Interacting Domain (SID®) polypeptide is firstly immobilised onto a suitable substrate, preferably a substrate of a kind which is generally used for manufacturing affinity chromatography substrates, most preferably immuno-affinity chromatography substrates which are well known from the one skilled in the art.

An illustrative example of such a suitable substrate consists of an agarose substrate.

The immobilisation of the Selected Interacting Domain (SID®) polypeptide onto the substrate is generally performed by conventional chemical coupling of said Selected Interacting Domain (SID®) polypeptide onto said substrate.

For the purpose of purifying a polypeptide of interest which is suspected, or alternatively which is known, to be contained in a sample, said sample is brought into contact with the substrate onto which a Selected Interacting Domain (SID®) polypeptide or a plurality of Selected Interacting Domain (SID®) polypeptides which bind specifically to said polypeptide of interest are immobilised.

Thus, the present invention also concerns a method for purifying a polypeptide of interest from a sample, wherein said method comprises the steps of :
a) providing a substrate onto which is immobilised a Selected Interacting Domain (SID®) polypeptide or a variant thereof which binds specifically to said polypeptide of interest;
b) bringing into contact the sample containing said polypeptide of interest with the substrate provided in step a).
   Preferably, the Selected Interacting Domain (SID®) polypeptide or a variant thereof is covalently bound to said substrate.
   The purification method above may further comprise the steps of:
c) removing the portion of the sample which is not bound to the immobilized Selected Interacting Domain (SID®) polypeptide; and
d) removing the polypeptide of interest from the substrate.

Accordingly, the removing of the polypeptide of interest from the substrate is performed in conventional conditions which are also used in the art for eluting a polypeptide which has been previously selectively retained onto an immunoaffinity chromatography column.

The invention further relates to a device for purifying a polypeptide of interest contained in a sample, wherein said device comprises a substrate onto which a Selected Interacting Domain (SID®) polypeptide which specifically binds to said polypeptide of interest is bound.

Preferably, the Selected Interacting Domain (SID®) polypeptide is covalently bound to said substrate.

### PHARMACEUTICAL COMPOSITIONS CONTAINING A SELECTED INTERACTING DOMAIN (SID®) POLYPEPTIDE.

It flows from the method according to which a Selected Interacting Domain (SID®) polypeptide has been selected and
characterized that such a Selected Interacting Domain (SID®) polypeptide or a variant thereof is both:
(i) endowed with highly specific binding properties to a (bait) polypeptide of interest; and
(ii) devoided of the biological activity of the naturally occurring protein from which this Selected Interacting Domain (SID®) polypeptide or a variant thereof is derived.

These original properties of a Selected Interacting Domain (SID®) polypeptide or a variant thereof allow its use for interfering with a naturally occurring interaction between a first protein and a second protein within the cell of an organism by the binding of said Selected Interacting Domain (SID®) polypeptide specifically either to said first polypeptide or said second polypeptide.

Thus, another object of the invention consists of a pharmaceutical composition comprising a pharmaceutically effective amount of a Selected Interacting Domain (SID®) polypeptide or a variant thereof.

The invention also relates to a pharmaceutical composition comprising a pharmaceutically effective amount of a nucleic acid comprising a polynucleotide encoding a Selected Interacting Domain (SID®) polypeptide or a variant thereof which polynucleotide is placed under the control of an appropriate regulatory sequence.

The invention also pertains to a pharmaceutical composition comprising a pharmaceutically effective amount of a recombinant expression vector comprising a polynucleotide encoding the Selected Interacting Domain (SID®) polypeptide or a variant thereof.

In a first preferred embodiment of a pharmaceutical composition as described above, the Selected Interacting Domain (SID®) polypeptide binds to a viral or a bacterial polypeptide.

In a second preferred embodiment of a pharmaceutical composition of the invention, the Selected Interacting Domain (SID®) polypeptide binds to a Helicobacter pylori polypeptide.

The invention also pertains to a method for preventing or curing a viral or a bacterial infection in a human or an animal, wherein said method comprises a step of administering to the human or animal body a pharmaceutically effective amount of a Selected Interacting Domain (SID®) polypeptide which binds to a targeted viral or bacterial protein.

A pharmaceutical composition as described above, wherein said composition is administered by any route, such as intravenous route, intramuscular route, oral route, or mucosal route with an acceptable physiological carrier and/or adjuvant, also forms part of the invention.

The Selected Interacting Domain (SID®) polypeptide or a variant thereof as a medicament for the prevention and/or treatment of pathologies of infection diseases induced by prokaryotic micro-organism are preferred in particular.

A most preferred are the Selected Interacting Domain (SID®) polypeptides that are used for the prevention and/or treatment of infection diseases induced by Helicobacter pylori.

The Selected Interacting Domain (SID®) polypeptides as active ingredients of a pharmaceutical composition will be preferably insoluble form combined with a pharmaceutically acceptable vehicle.

Such compounds which can be used in a pharmaceutical composition offer a new approach for preventing and/or treating pathologies linked to infection by prokaryotic micro-organism such as Helicobacter pylori. Preferably, these compounds will be administered by the systemic route, in particular by the intravenous route, by the intramuscular or intradermal route or by the oral route.

Their modes of administration, optimum dosages and galenic forms can be determined according to the criteria generally taken into account in establishing a treatment suited to a patient, such as for example the age or body weight of the patient, the seriousness of his general condition, the tolerance to treatment and the side effects observed, and the like.

The identified compound can be administered to a mammal, including a human patient, alone or in pharmaceutical compositions where they are mixed with suitable carriers or excipients at therapeutically effective doses to treat disorders associated with prokaryotic micro-organism infection. Techniques for formulation and administration of the compounds of the invention may be found in « Remington's Pharmaceutical Sciences » Mack Publication Co., Easton, PA, latest edition.

For any Selected Interacting Domain (SID®) polypeptide or any variant thereof used according to the invention, the therapeutically effective dose can be estimated initially from cell culture assays. For example, a dose can be formulated in animal models to achieve a circulating concentration range that includes or encompasses a concentration point or range shown the desired effect in an *in vitro* system. Such information can be used to more accurately determine useful doses in humans.

A therapeutically effective dose refers to that amount of the compound that results in amelioration of symptoms in a patient. Toxicity and therapeutic efficacy of such compounds can be determined by standard pharmaceutical procedures in cell cultures or experimental animals, e.g. for determining the LD50, (the dose lethal to 50% of the test population) and the ED50 (the dose therapeutically effective in 50% of the population). The dose ratio between toxic and therapeutic effects is the therapeutic index and it can be expressed as the ratio between LD50 and ED50 Compounds which exhibit high therapeutic indices are preferred.

The data obtained from these cell culture assays and animal studies can be used in formulating a range of dosage for use in human. The dosage of such compounds lies preferably within a range of circulating concentrations that include the ED50, with little or no toxicity. The dosage may vary within this range depending upon the dosage form employed and the route of administration utilised. The exact formulation, route of administration and dosage can be chosen by the individual physician in view of the patient's condition. (See, e.g. FingI et al. 1975, in « The Pharmacological Basis of Therapeutics », CH.I).

Dosage amount and interval may be adjusted individually to provide plasma levels of the active compound which are sufficient to maintain the modulating effects. Dosages necessary to achieve the modulating effect will depend on individual characteristics and route of administration.

The amount of composition administered will, of course, be dependent on the subject being treated, on the subject's weight, the severity of the affliction, the manner of administration and the judgement of the prescribing physician.

The invention also pertains to a method for preventing or curing a viral or bacterial infection in a human or an animal, wherein said method comprises the step of administering to the human or animal body a pharmaceutically effective amount of a nucleic acid comprising a polynucleotide encoding a Selected Interacting Domain (SD®) polypeptide which binds to a viral or a bacterial protein, and wherein said polynucleotide is placed under the control of a regulatory sequence which is functional in said human or said animal.

The invention also relates to a method for preventing or curing a viral or a bacterial infection in a human or an animal, wherein said method comprises the step of administering to the human or animal body a pharmaceutically effective amount of a recombinant expression vector comprising a polynucleotide encoding a Selected Interacting Domain (SD®) polypeptide which binds to a viral or bacterial protein.

Other characteristics and advantages of the invention appear in the remainder of the description with the examples below.

### BIBLIOGRAPHIC REFERENCES:

- Aguzzi F et al. , Estratto Dal. Boll. 1 st Sieroter; Milanese, 1977, vol.56: 212-216.
- Adams et al., (1995), FASEB J., vol.9, (6):A 1336, Abstract N°465
- Adams et al., 1985, Nature 318:533-538.
- Benoist and Chambon, 1981, Nature 290:304-310.
- Brinster et al., 1982, Nature 296:39-42.
- Brigham KL et al., 1993, Am. J. Respir Cell Mol. Biol.8(2):209-213
- Behr, 1994, Bioconjugate Chem., 5:382-389
- Benoist and Chambon, 1981, Nature 290:304-310.
- Brinster et al., 1982, Nature 296:39-42.
- Curiel et al. Gene Transfer to Respiratory Epithelial Cells via the Receptor Mediated Endocytosis Pathway, Am. J. Respir. Cell Mol. Biol. 6 (1992) 247-252
- Curiel et al. Adenovirus Enhancement of Transferrin-Polylysine-Mediated Gene Delivery, Proc. Natl. Acad. Sci. 88 (1991) 8850-8854
- Chen F-T.A. et al. , 1991, Clin. Chem., vol.77: 14-19.
- Chalfie et al., (1994), Science, vol. 263: 802-805.
- Davis et al., (1995), Development Biology, vol.170: 726-729
- Drumm, M. L. et al., Cell 62:1227-1233 (1990).
- Delagrave et al., (1995), Biotechnology, vol.13: 151-154
- DeBoer, et al., 1983, Proc. Natl. Acad. Sci. U.S.A. 80:21-25.
- Felgner, P. L., Gadek, T. R., Holm, M., Roman, R., Chan, H. W., Wenz, M., Northrop, J. P., Ringold, G. M. and Danielsen, M. 1987. Lipofection: a highly efficient, lipid-mediated DNA-transfection procedure. Proc. Natl. Acad. Sci. U.S.A. 84(21): 7413-7
- FINGL et al. 1975, in «The Pharmacological Basis of Therapeutics », CH.I
- Fromont-racine M et al. (1997), Nature Genetics, vol.16 (3):277-282
- Flajolet et M. et al. (2000), Gene, vol.242:369-379
- Goding et al. J.W., (1986), In: Monoclonal antibodies: Principles and practice-production and application of monoclonal antibodies in cell biology, biochemistry and immunology, Acad. Press, London, pages 255-280.
- Grosschedl et al., 1984, Cell 38:647-658.
- Handl et al., 1988, J.Clin. Microbiol., vol.26: 1555-1560
- Hanahan, 1985, Nature 315:115-122.
- Heim et al., (1994), Proc. Natl. Acad. Sci., volume 91: 12501-12.504.
- Heim et al., (1995), Nature, vol. 373: 663-664.
- Horisberger, (1981), Scanning electron microscopy, pages 19-40.
- Hu and Cheng, (1995), Febs. Letters, vol.369: 331-334.
- Krumlauf et al., 1985, Mol. Cell. Biol. 5:1639-1648.
- Kaufman, 1991, *Current Protocols in Molecular Biology,* 16.12
- KOCH Y, 1977, Biochem. Biophys. Res. Commun, vol.74:488-491
- Leder et al., 1986, Cell 45:485-495.
- Mason et al., 1986, Science 234:1372-1378.
- Macdonald, 1987, Hepatology 7:425-515.
- Maggio ET, « Enzyme-immuno assay », 1980, CRC Press Incorporated, Boca Raton, Fla.
- Merlini G et al. , 1983, J. Clin. Chem. Biochem. , vol.21: 841-844,
- Muzyczka, N., Curr. Top. Micro. Immuno. 158:97-129 (1992)
- Nielsen et al., 1991, J. Chromatogr., vol.539: 177
- Prascher DC et al., 1992, Gene, 229-233
- Peterhans et al., (1987), Analytical biochemistry, vol. 63:470-475.
- Ichinose N et al;, (1991), In: Fluorometric analysis in biomedical chemistry, vol.10, page 110, Chemical analysis, Winefordner JD et al. Eds., John Wiley and Sons, New York.
- Keegan et al. (1986), Science, vol.231 (4739): 699-704.
- Kaether and Gerdes, (1995), Febs. Letters, vol.369:267-271.
- Kollias et al., 1986. Cell 46:89-94.
- Kohler and Milstein, 1975, Nature, 256: 495
- Kozbor et al., 1983, Hybridoma, 2(1):7-16.
- Leger et al., 1997, Hum. Antibodies, 8(1):3-16
- Martineau et al., 1998, J. Mol. Biol., 280(1): 117-127
- Muzyczka N., 1992, Curr. Top. Micro. Immuno, 158: 97-129
- Mogram et al., 1985, Nature 315:338-340.
- Martin et al., (1990), Characterization of antibody labelled colloïdal gold particles and their applicability in a sol particle immunoassay, SPIA, J. Immunoassay, vol.11:31-48.
- Pinkert et al., 1987, Genes and Devel. 1:268-276.
- Pontiroli et al., 1987, Diabet. Metab., vol.13:441-443.
- Plank et al. Gene Transfer into Hepatocytes Using Asialoglycoprotein Receptor Mediated Endocytosis of DNA Complexed with an Artificial Tetra-Antennary Galactose Ligand Bioconj. Chem. 3 (1992) 533-539
- Rosenfeld, M. A. et al., Cell 68:143-155 (1992)
- Rizzuto et al. , (1995), Current Biology, vol.5: 635-142.
- Ridder et al., 1995, Biotechnology (NY), 13(3):255-260
- Reinmann et al., 1997, AIDS Res. Hum. Retroviruses, 13(II): 933-943
- Readhead et al., 1987, Cell 48:703-712.
- Sternberg, B., Sorgi, F. L. and Huang, L. 1994. New structures in complex formation between DNA and cationic liposomes visualized by freeze-fracture electron microscopy. FEBS. Lett. 356(2-3): 361-6
- Sternberg B. etal., 1994, FEBS Letters, 356(2-3):361-366
- Schofield, Brit. Microencapsulated. Bull., 51(1):56-71 (1995) Behr, Bioconjugate Chem., 5, 382-389 (1994)
- Sani, 1985, Nature 314:283-286.
- Shattil SJ et al., (1987), Blood, vol.70: 307.
- Shattil et al. SJ(1985), J. Biol. Chem., vol.260:11.107.
- Swift et al., 1984, Cell 38:639-646.
- Smith *et al*., 1988, Gene 67:31-40.
- Trubetskoy, V. S. et al., Biochem. Biophys. Acta 1131:311-313 (1993))
-
- Villa-koumaroff, et al., 1978, Proc. Natl. Acad. Sci. U.S.A. 75:3727-3731.
- Wagner et al. Influenza Virus Hemagglutin HA2 N-Terminal Fusogenic Peptides Augment Gene Transfer by Transferrin-Polylysine-DNA Compleses: Toward a Synthetic Virus-like Gene-Transfer Vehicle, Proc. Natl. Acad. SC4. 89 (1992) 7934-7938
- Wilson, J. M. et al., 1992, Endocrinology, 130(5):2947-2954
- White Wa et al., 1986, Biochem. Clin. vol.10:571-574.
- Wrobel, I. and Collins, D. 1995. Fusion of cationic liposomes with mammalian cells occurs after endocytosis. Biochim. Biophys. Acta 1235(2): 296-304
- Wu et al., 1992, J. Biol. Chem. 267:963-967.
- Wu and Wu, 1987. J. Biol. Chem. 262:4429-4432.
- Wu and Wu, 1988, J. Biol. Chem. 263:14621-14624.
- YAMAMOTO, et al., 1980, Cell 22:787-797.
- Zabner, J., Fasbender, A. J., Moninger, T., Poellinger, K. A. and Welsh, M. J. 1995. Cellular and molecular barriers to gene transfer by a cationic lipid. J. Biol. Chem. 270(32): 18997-9007
-

## Claims

1. A marker compound wherein said compound comprises :
a) a Selected Interacting Domain (SID®) polypeptide or a variant thereof that binds specifically to the polypeptide of interest; and
b) a detectable molecule bound thereto.

2. The marker compound of claim 1, wherein the detectable molecule consists of a fluorescent protein.

3. The marker compound of claim 4, wherein the detectable protein is selected from the group consisting of GFP and YFP.

4. The marker compound of claim 1, wherein the detectable molecule is endowed with a catalytic activity.

5. The marker compound of claim 4, wherein the detectable molecule is selected from the group consisting of a hydrolase, a transferase, a lyase, an isomerase, a ligase, a synthetase and a oxidoreductase.

6. The marker compound of claim 1, wherein the detectable molecule is radioactive.

7. The marker compound of claim 1, wherein the detectable molecule is chemiluminescent.

8. The marker compound according to any one of claims 1 to 7, wherein the detectable molecule is covalently bound to the Selected Interacting Domain (SID®) polypeptide or a variant thereof.

9. The marker compound according to any one of claims 1 to 7, wherein the detectable molecule is non covalently bound to the Selected Interacting Domain (SID®) polypeptide or a variant thereof.

10. The marker compound of claim 9, wherein the detectable molecule is an antibody directed specifically against the Selected Interacting Domain (SID®) polypeptide.

11. The marker compound of claim 10, wherein said antibody is labelled radioactively or non radioactively.

12. The marker compound according to claim 1, wherein :
a) the Selected Interacting Domain (SID®) polypeptide of a variant thereof onto which is covalently bound a first ligand.; and
b) the detectable molecule comprises a second ligand which binds specifically to the first ligand

13. The marker compound according to claim 12, wherein the first ligand is biotin and the second ligand is streptavidin.

14. A nucleic acid encoding a marker compound according to any one of claims 1 to 8.

15. A nucleic acid encoding the Selected Interacting Domain (SID®) polypeptide or a variant thereof onto which is covalently bound a first ligand defined in claims 12 and 13.

16. A recombinant vector comprising inserted therein a nucleic acid according to any one of claims 14 and 15.

17. The recombinant vector according to claim 16, which is selected from the group consisting of pACTIIst, pASΔΔ, pT25, pKT25, pUT18 and pUT18C.

18. A recombinant cell host which has been transfected with a nucleic acid according to any one of claims 14 and 15 or a recombinant vector according to any one of claims 16 and 17.

19. The recombinant cell host according to claim 18 which is of prokaryotic origin.

20. The recombinant cell host according to claim 18 which is of eukaryotic origin.

21. The recombinant cell host according to claim 18 which is a mammalian cell host.

22. A method for detecting a polypeptide of interest within a sample, which comprises the steps of:
a) contacting a marker compound or a plurality of marker compounds according to any one of claims 1 to 13 with the sample;
b) detecting the complexes formed between said marker compound or said plurality of marker compounds and said polypeptide of interest.

23. The method of claim 22; wherein the sample is a biological fluid.

24. The method of claim 22, wherein the sample is a cell culture.

25. The method of claim 22, wherein the sample is a tissue section.

26. The method of claim 22, wherein the marker compound or the plurality of marker compounds is (are) immobilised onto a substrate.

27. The method of claim 22, wherein the detection step b) consists of the measure of the fluorescence signal intrinsically emitted by the detectable molecule.

28. The method of claim 22, wherein the detection step b) consists of submitting the detectable molecule to a source of energy at the excitation wavelength and measuring the light emitted at the emission wavelength of said detectable molecule.

29. The method of claim 22, wherein the detection step b) consists of measuring the catalytic activity of the detectable molecule.

30. The method of claim 22, wherein the detection step b) consists of measuring the radioactivity emitted by the detectable molecule.

31. A kit for detecting a polypeptide of interest within a sample, which comprises a marker compound according to any one of claims 1 to 13.

32. The kit of claim 31 which further comprises the reagents necessary to carry out the detection step b).

33. A method for detecting a polypeptide of interest within a prokaryotic or an eukaryotic cell host, said method comprising the steps of :
a) providing a cell host to be assayed;
b) transfecting said cell host with a nucleic acid according to any one of claims 14 and 15 or with a recombinant vector according to any one of claims 16 and 17;
c) detecting the complexes formed between the marker compound expressed by the transfected cell host and the polypeptide of interest.

34. The method of claim 33, wherein the detection step c) consists of the measure of the fluorescence signal intrinsically emitted by the detectable molecule.

35. The method of claim 33, wherein the detection step c) consists of submitting the detectable molecule to a source of energy at the excitation wavelength and measuring the light emitted at the emission wavelength of said detectable molecule.

36. The method of claim 33, wherein the detection step c) consists of measuring the catalytic activity of the detectable molecule.

37. The method of claim 33 wherein the detection step c) consists of measuring the radioactivity emitted by the detectable molecule.

38. The method of claim 33, wherein detection step c) allows the location of the complexes formed between the marker compound and the polypeptide of interest within the transfected cell host.

39. A kit for detecting a polypeptide of interest within a prokaryotic or an eukaryotic cell host which comprises a nucleic acid according to any one of claims 14 and 15 or a recombinant vector according to any one of claims 16 and 17.

40. The kit of claim 39 which further comprises the reagents necessary to carry out the detection step c).

41. A method for detecting a polypeptide of interest within a prokaryotic or eukaryotic cell host, said method comprising the steps of:
a) providing a cell host to be assayed;
b) introducing a marker compound according to any one of claims 1 to 11 within said cell host;
c) detecting the complexes formed between the marker compound and the polypeptide of interest within the cell.

42. The method of claim 41, wherein the detection step c) consists of the measure of the fluorescence signal intrinsically emitted by the detectable molecule.

43. The method of claim 41, wherein the detection step c) consists of submitting the detectable molecule to a source of energy at the excitation wavelength and measuring the light emitted at the emission wavelength of said detectable molecule.

44. The method of claim 41, wherein the detection step c) consists of measuring the catalytic activity of the detectable molecule.

45. The method of claim 41, wherein the detection step c) consists of measuring the radioactivity emitted by the detectable molecule.

46. The method of claim 41, wherein detection step c) allows the location of the complexes formed between the marker compound and the polypeptide of interest within the transfected cell host.

47. A kit for detecting a polypeptide of interest within a prokaryotic or eukaryotic cell host comprising a marker compound according to any one of claims 1 to 13.

48. The kit of claim 47 which further comprises the reagents necessary to carry out the detection step c).

49. The kit of claim 47 which further comprises the reagents necessary to facilitate the introduction of the marker compound within the cell host.

50. A method for detecting a polypeptide or a plurality of polypeptides of interest within a sample, wherein said method comprises the steps of:
a) providing a substrate onto which a Selected Interacting Domain (SID®) polypeptide or a variant thereof, or a plurality of Selected Interacting Domain (SID®) polypeptides or variants thereof is (are) immobilised.
b) bringing into contact the substrate defined in a) with the sample to be assayed;
c) detecting the complexes formed between the Selected Interacting Domain (SID®) polypeptides or variants thereof or a variant thereof, or the plurality of Selected Interacting Domain (SID®) polypeptide and a molecule or a plurality of molecules initially contained in the sample;

51. The method of claim 50, wherein a plurality of Selected Interacting Domain (SID®) polypeptides or variants thereof are immobilised on the substrate in an ordered manner.

52. The method of claim 50, wherein the Selected Interacting Domain (SID®) polypeptide or a variant thereof, or the plurality of Selected Interacting Domain (SID®) polypeptides or variants thereof are covalently bound to the substrate.

53. The method of claim 50, wherein the Selected Interacting Domain (SID®) polypeptide or a variant thereof, or the plurality of Selected Interacting Domain (SID®) polypeptides or a variant thereof are non- covalently bound to the substrate.

54. The method of claim 53, wherein the Selected Interacting Domain (SID®) polypeptide or a variant thereof, or the plurality of Selected Interacting Domain (SID®) polypeptides or variants thereof are covalently bound to a first ligand and wherein the substrate is coated with a second ligand which specifically binds to the first ligand.

55. The method of claim 54, wherein the first ligand is biotin and the second ligand is streptavidin.

56. The method according to any one of claims 50 to 55, wherein the Selected Interacting Domain (SID®) polypeptide or a variant thereof, or the plurality of Selected Interacting Domain (SID®) polypeptides or variants thereof are covalently linked to a spacer and wherein said spacer is covalently bound to the substrate in order to immobilise the Selected Interacting Domain (SID®) polypeptide, or a variant thereof or the plurality of Selected Interacting Domain (SID®) polypeptides.

57. The method according to any one of claims 50 to 55, wherein the detection step c) consists of detecting changes in optical characteristics of the substrate.

58. A device for the detection of a polypeptide or a plurality of polypeptides of interest within a sample, wherein said device comprises a substrate onto which a Selected Interacting Domain (SID®) polypeptide or a variant thereof or a plurality of Selected Interacting Domain (SID®) polypeptides or variants thereof is (are) immobilised.

59. The use of a Selected Interacting Domain (SID®) polypeptide or a variant thereof for the specific detection of a polypeptide of interest within a sample.

60. A method for labelling a polypeptide of interest, wherein said method comprises the steps of :
a) providing a Selected Interacting Domain (SID®) polypeptide which binds specifically to said polypeptide of interest;
b) bringing into contact the Selected Interacting Domain (SID®) polypeptide with said polypeptide of interest.

61. A method for labelling a polypeptide of interest, wherein said method comprises the steps of :
a) providing a marker compound according to anyone of claims 1 to 13, which binds specifically to said polypeptide of interest;
b) bringing into contact said marker compound with said polypeptide of interest.

62. A method for purifying a polypeptide of interest from a sample, wherein said method comprises the steps of:
a) providing a substrate onto which is immobilised a Selected Interacting Domain (SID®) polypeptide or a variant thereof which binds specifically to said polypeptide of interest;
b) bringing into contact the sample containing said polypeptide of interest with the substrate onto which said Selected Interacting Domain (SID®) polypeptide has been immobilized.

63. The method of claim 62, wherein the Selected Interacting Domain (SID®) polypeptide or a variant thereof is covalently bound to the substrate.

64. The method of claim 62, which further comprises the steps of:
c) removing the portion of the sample which is not bound to the immobilized Selected Interacting Domain (SID®) polypeptide; and
d) remaining the polypeptide of interest from the substrate.

65. A device for purifying a polypeptide of interest contained in a sample, wherein said device comprises a substrate onto which a Selected Interacting Domain (SID®) polypeptide which specifically binds to said polypeptide of interest is bound.

66. A pharmaceutical composition comprising a pharmaceutically effective amount of a Selected Interacting Domain (SID®) polypeptide or a variant thereof.

67. A pharmaceutical composition comprising a pharmaceutically effective amount of a nucleic acid comprising a polynucleotide encoding a Selected Interacting Domain (SID®) polypeptide or a variant thereof, which polynucleotide is placed under the control of an appropriate regulatory sequence.

68. A pharmaceutical composition comprising a pharmaceutically effective amount of a recombinant expression vector comprising a polynucleotide encoding a Selected Interacting Domain (SID®) polypeptide or a variant thereof.

69. A pharmaceutical composition according to anyone of claims 66 to 68 wherein the Selected Interacting Domain (SID®) polypeptide binds to a viral or a bacterial polypeptide.

70. A pharmaceutical composition according to claim 69 wherein the Selected Interacting Domain (SID®) polypeptide binds to a Helicobacter pylori polypeptide.

71. A method for preventing or curing a viral or a bacterial infection in a human or an animal, wherein said method comprises a step of administering to the human or animal body a pharmaceutically effective amount of a Selected Interacting Domain (SID®) polypeptide which binds to a targeted viral or a bacterial protein.

72. A method for preventing or curing a viral or a bacterial infection in a human or an animal, wherein said method comprises a step of administering to the human or animal body a pharmaceutically effective amount of a nucleic acid comprising a polynucleotide encoding a Selected Interacting Domain (SID®) polypeptide which binds to a viral or a bacterial protein, and wherein said polynucleotide is placed under the control of regulatory sequence which is functional in said human or said animal.

73. A method for preventing or curing a viral or a bacterial infection in a human or an animal, wherein said method comprises a step of administering to the human or animal body a pharmaceutically effective amount of a recombinant expression vector comprising a polynucleotide encoding a Selected Interacting Domain (SID®) polypeptide which binds to a viral or a bacterial protein.
